# EUROPÄISCHE PATENTSCHRIFT

(11) **EP 1 737 795 B1**
(45) Veröffentlichungstag und Bekanntmachung des Hinweises auf die Patenterteilung: **15.01.2014**
(21) Anmeldenummer: 05732390.9
(22) Anmeldetag: 11.04.2005
(51) Int. Cl.: C02F 1/32, B01D 53/00, B01J 19/12

(54) **VORRICHTUNG ZUR BEHANDLUNG EINES FLÜSSIGEN ODER GASFÖRMIGEN MEDIUMS MITTELS UV-STRAHLEN**
DEVICE FOR THE TREATMENT OF A LIQUID OR GASEOUS MEDIUM BY MEANS OF UV RADIATION
DISPOSITIF POUR TRAITER UNE SUBSTANCE LIQUIDE OU GAZEUSE AU MOYEN DE RAYONS UV

(30) Priorität: 13.04.2004 EP 04008805; 20.12.2004 DE 102004061253
(43) Veröffentlichungstag der Anmeldung: 03.01.2007
(73) Patentinhaber: Araiza, Rafael, 6318 Walchwil (CH); Hartig, Heinz, 81373 München (DE)
(72) Erfinder: Araiza, Rafael, 6318 Walchwil (CH); Hartig, Heinz, 81373 München (DE)
(74) Vertreter: von Puttkamer, Nikolaus
(86) Internationale Anmeldenummer: PCT/EP2005/003776
(87) Internationale Veröffentlichungsnummer: WO 2005/100256

(56) Entgegenhaltungen:
- WO-A-89/02418
- WO-A-95/13853
- DE-A1- 4 233 566
- US-A- 3 519 817
- US-A- 3 767 918
- US-A- 4 968 437
- US-A- 5 597 482

## Beschreibung

### TECHNISCHES GEBIET

Die vorliegende Erfindung betrifft eine Vorrichtung zur Behandlung eines flüssigen oder gasförmigen Mediums mittels ultravioletter strahlen (UV-Strahlen). Ferner betrifft die Erfindung eine besonders geeignete Verwendung dieser Vorrichtung.

### STAND DER TECHNIK

As UV-Strahlung bezeichnet man die elektromagnetische Strahlung, die zwischen der sichtbaren Grenze von kurzwelligem Licht und Röntgenstrahlung angesledelt ist und In einem Wellenlängenbereich von ca. 100 nm bis 400 nm liegt. Man unterscheldet folgenden Bereiche: UVA-Strahlung: 400 - 315 nm; UVB-Strahlung: 315 - 280 nm; UVC-Strahlung: ca. 280 - 200 nm; und VUV-Strahlung: ca. < 180 nm. Vorrichtungen zur Behandlung eines flüssigen oder gasförmigen Mediums mittels UV-Strahlen werden zum Beispiel zur UV-Entkeimung oder zur UV-Oxidation eingesetzt.

Bei der UV-Entkeimung wird in der Regel UVC-Strahlung verwendet, welche Mikroorganismen wie Bakterien, Hefen und Pilze durch Schädigung der DNS (Desoxiribonucleinsäure) wirksam abgetötet. Eine Wellenlänge der UV-Strahlung in einem Bereich von ca. 260 nm bis 280 nm, insbesondere bei ca. 254 nm, erzielt hierbei eine besonders hohe Entkeimungswirkung, da das Absorbtionsspektrum der DNS bei ca. 254 nm ein Maximum besitzt. Durch die hohe Absorption der DNS In diesem UV-Strahlungsbereich wird eine fotochemische Reaktion ausgelöst, die in den bestrahlten Mikroorganismen eine Unterbrechung der genetischen Information zur Zellvermehrung und für den Stoffwechsel bewirkt. Die Mikroorganismen werden auf diese Weise inaktiviert und unschädlich gemacht. Eine Zugabe von Chemikalien ist bei der UV-Entkeimung nicht erforderlich.

Bei Wellenlängen unter 230nm ist die Energie der UV-Strahlung bereits ausreichend, um chemische Bindungen aufzubrechen. UV-Strahlung mit Wellenlängen kleiner 200 nm beinhaltet ionisierende Strahlen, die fotochemische Oxidationsprozesse auslösen, die zur Reinigung von Gasen oder Flüssigkeiten, wie z.B. Abwässern, genutzt werden können, die mit organischen Schadstoffen, wie z.B. Pflanzenschutzmitteln, Hormonen, Dioxinen, Medikamentresten, etc. kontaminiert sind. Bei dieser UV-Oxidation werden die Moleküle der organischen Substanzen oxidiert und zu ungiftigen Verbindungen abgebaut. Dieses Verfahren wird auch als photochemische Nassverbrennung bezeichnet. Für industrielle Anwendungen wird dem Abwasser noch ein separates Oxidationsmittel (z.B. H₂O₂, Ozon) beigegeben. Durch die UV-Strahlung werden die oxidierbaren Substanzen im Abwasser mineralisiert. Zusätzlich wird das Oxidationsmittel in hoch reaktive Radikale gespalten. Diese Radikale tragen ebenfalls zur vollständigen Oxidation der unerwünschten Abwasserinhaltstoffe bel.

Aus der EP 0 470 518 A1 ist eine in Form eines fotochemischen Durchflussreaktors ausgestaltete Vorrichtung zur Behandlung eines flüssigen oder gasförmigen Mediums mittels UV-Strahlen bekannt. Diese zur UV-Oxidation verwendete Vorrichtung arbeitet vorzugsweise unter Verwendung eines zusätzlich zugeführten Oxidationsmittels. Die Vorrichtung umfasst eine UV-Strahlungsquelle mit einer axialen Längserstreckung und einer dazu im Wesentlichen senkrechten, radialen Abstrahlungsrichtung. Ferner besitzt die Vorrichtung eine einzeine Behandlungskammern in Gestalt eines langen Edelstahlbehälters, in welchem die UV-Strahlungsquelle angeordnet ist. Die Innenwand des Edelstahlbehälters ist poliert und dient als Reflektor für die UV-Strahlung. An der Innenwand des Edelstahlbehälters sind Verwirbelungselemente angebracht, welche das durchströmende Medium verwirbeln und vermischen und In den Wirkungsbereich der UV-Strahlungsquelle leiten sollen.

Aus der WO 89/02418A ist ein Verfahren und eine Vorrichtung zum Reinigen einer mit organischen und/oder anorganischen Stoffen belasteten Flüssigkeit offenbart. Die Fremdstoffe sollen dabei unter Ausnutzung der katalytischen und fotokatalytischen Eigenschaften von Halbleitern und gegebenenfalls unter Zugabe von Oxidationsmitteln abgeschieden werden. Durch eine Lichtanregung werden an der Oberfläche der Halbleiterschicht Redoxreaktionen ausgelöst.

Die DE 42 33 566 A1 offenbart einen Reaktor zum Reinigen oder Regenerieren von Flüssigkeiten, die mit Schadstoffen belastet sind. In einem Glasrohr ist mindestens ein Strahler aufgenommen, der im wesentlichen UV-Strahlung abstrahlt. Um den Strahler herum ist eine Vielzahl von Strömungsrohren aus Glas angeordnet. Die Strömungsrohre werden von der schadstoffbefasteten Flüssigkeit durchströmt. Die Schadstoffmoleküle werden durch die absorbierte Strahlung aufgebrochen.

Aus einer US A-3 767 918 ist ein Strahler für fließfähige Materialien bekannt. Zum Transport des fließfähigen Materials ist im Strahler ein einziger Strömungskanal vorgesehen. Die Strömungsrichtung innerhalb des Strömungskanals wird wiederholt umgekehrt.

Ferner sind aus der US A-3 519 817 ein Verfahren und eine Vorrichtung bekannt, bei denen kontinuierlich strömende Flüssigkeiten mithilfe einer radioaktiven Strömungsquelle bestrahlt werden. Vor dem Eintritt der Flüssigkeit in das Strahlungsfeld wird in periodischen Zeitintervallen ein Kolben in den Strömungsweg eingeführt. Der Kolben wird nach Verlassen des Strahlungsfeldes wieder aus dem Strömungsweg ausgeführt.

Zur Erzielung einer hinreichenden Entkeimungs- oder Oxidationswirkungsgrades sowie zur Erreichung eines ausreichenden Durchsatzes müssen konventionelle Vorrichtungen zur Behandlung eines flüssigen oder gasförmigen Mediums mittels UV-Strahlen eine erhebliche Baugröße aufweisen. Diese Baugröße erfordert nicht nur einen erheblichen Platzbedarf, sondem bedingt auch, dass die betreffende Vorrichtung nur mit relativ geringen Drücken arbelten kann, da andernfalls Festigkeitsprobleme auftreten. Darüber hinaus ist bei vorbekannten Vorrichtungen eine sehr hohe Leistung der UV-Strahlungsquelle erforderlich. Bei konventionellen Vorrichtungen, die zur UV-Entkeimung verwendet werden, hat es sich ferner gezeigt, dass oftmals ein Tell der Mikroorgarnismen in dem zu behandelnden Medium nicht voliständig inaktiviert wird. Darüber hinaus ist festzustellen, dass selbst bei einer vollständigen Inaktivierung der Mikroorganismen durch Zerstörung von deren DNS die Zellhülle der Mikroorganismen sowie deren Zusammensetzung weitgehend intakt bleibt. Dies kann bei Personen, die Unverträglichkelten gegen bestimmte Proteine oder chemische Zusammensetzungen aufweisen, nachteilig sein. Im Falle einer unvollständigen UV-Oxidation wiederum können organische Schadstoffe in dem Medium verbleiben, was ebenfalls nicht erstrebenswert ist. Ferner wäre es wünschenswert, bei der UV-Oxidation möglichst weitgehend auf zusätzlich beizufügende Oxidationsmittel verzichten zu können.

### DARSTELLUNG DER ERFINDUNG

Der Erfindung liegt die Aufgabe beziehungsweise des technische Problem zugrunde, eine einfache und effektive Vorrichtung zur Behandlung eines flüssigen oder gasförmigen Mediums mittels UV-Strahlen zu schaffen, welche die dem Stand der Technik anhaftenden Nachtelle möglichst vermeidet. Gemäß einem weiteren Aspekt dieser Aufgabe soll eine besonders geeignete Verwendung für eine solche Vorrichtung aufgezeigt werden. Nach einem weiteren Aspekt der Aufgabe soll die Verweilzeit des Mediums in der UV-Strahlung erhöht werden.

Diese Aufgabe wird gemäß einem ersten Aspekt gelöst durch eine erfindungsgemäße Vorrichtung mit den Merkmalen des Anspruchs 1.

Diese Vorrichtung zur Behandlung eines flüssigen oder gasförmigen Mediums, Insbesondere Wasser oder Luft, mittels UV-strahlen, umfasst: eine UV-Strahlungsquelle mit einer axialen Längserstreckung und einer dazu im Wesentlichen senkrechten, Insbesondere radialen Abstrahlungsrichtung; und mehrere (d.h. mindestens zwei) Schichten von in Abstrahlungsrichtung übereinander angeordneten, aufeinanderfolgenden, durchstrahlbaren Behandlungskammern, die von der UV-Strahlungsquelle und voneinander jeweils durch eine transparente, UV-durchlässige Trennschicht getrennt sind, und die, beginnend bei einer der UV-Strahlungsquelle In Abstrahlungsrichtung nächstliegenden, ersten Behandlungskammer, jeweils einen entlang der Längserstreckung der UV-Strahlungsquelle verlaufenden Durchströmungskanal für das Medium bilden, der in die jeweils nachfolgende, von der UV-Strahlungsquelle in Abstrahlungsrichtung weiter entfernte Behandlungskammer mündet.

Die Erfindung sieht weiterhin vor, dass die transparente, UV-durchlässige Trennschicht als Bestandteile transparente und UV-durchlässige Verwirbelungselemente aufweist. Die transparenten und UV-durchlässigen Verwirbelungselemente sind jeweils als optische Linse ausgebildet

Die erfindungsgemäße Vorrichtung arbeitet im Durchflussverfahren. Die UV-Strahlungsquelle emittiert vorzugsweise UV-Strahlen Im UVC und/oder VUV-Bereich. Die UV-Strahlungsquelle kann eine einzige oder mehrere UV-Einzelstrahlungsquellen umfassen, die an einer gemeinsamen Stelle oder auch an unterschiedlichen Stellen der Vorrichtung angeordnet sind. Falls mehrere UV-Einzeistrahlungsquellen Anwendung finden, so ist es möglich, dass diese sowohl gleiche als auch unterschledliche UV-Strahlungspektren aufweisen. Beispielsweise kann dann eine UV-Einzelstrahlungsquellen im UVC- und die andere und VUV-Bereich strahlen. Je nach konkretem Anwendungsfall sind als UV-Strahlungsquelle Nieder-, Mittel- und Hochdruckstrahler einsetzbar. Die Anzahl der Schichten von Behandlungskammern kann insbesondere in Abhängigkeit der UV-Strahlungsquelle, des zu behandelnden Mediums und seiner Verunreinigungen variieren. Ferner können die Abmessungen und Geometrien der jeweiligen Behandlungskammern, insbesondere Ihre Dicke In Strahlungsrichtung bzw. Ihre Strömungsquerschnitte gleich oder unterschiedlich sein. In Verbindung mit dem jeweils zu behandeinden Medium ist dadurch eine gewünschte Strömungsdynamik sowie eine optimale Ausnutzung der Eindringtiefe der UV-Strahlung oder eines bestimmten Spektralbereichs in das Medium und ein bastimmte Absorptions- und Reaktionsverhalten des Mediums erreichbar. Auch die transparenten, UV-durchlässigen Trennschichten oder Teilbereiche davon können gleiche oder unterschiedliche Eigenschaften, insbesondere UV-Transmissionseigenschaften, und Abmessungen bzw. Dicken aufweisen.

Die erfindungsgemäße Vorrichtung ist gegenüber konventionellen gattungsgemäßen Vorrichtungen vergleichsweise klein, kompakt und mit einem niedrigen Bauvolumen ausführbar und benötigt nur einen geringen Platzbedarf. Dennoch ist sie sehr robust. Die kompakte Bauweise ermöglicht es auch, mit höheren Drücken als beim Stand der Technik zu arbeiten, wodurch der Durchsatz des Mediums erhöht bzw. mit einer kleineren Vorrichtung ein gleicher Durchsatz wie bei einer größeren vorbekannten Vorrichtung erzielt werden kann. Während vorbekannte Vorrichtungen beispielsweise mit ca. 1,5 bar arbeiten, so ist die erfindungsgemäße Vorrichtung z.B. ohne Weiteres mit 4,5 bar betreibbar. Durch die geschichtete bzw. mehrfach geschichtete Anordnung der Behandlungskammern lässt sich trotz der geringen Baugröße die Verweilzeit des zu behandelnden Mediums und der darin enthaltenen Verunreinigungen im Wirkungsbereich der UV-Strahlungsquelle erheblich erhöhen. Infolge der erfindungsgemäßen Bauweise wird hierbei der Strahlungsbereich der UV-Strahlungsquelle optimal genutzt und die Einwirkungsdauer der UV-Strahlen auf kleinstem Raum maximiert, da das zu behandelnde Medium während eines einzigen Durchlaufs durch die Vorrichtung mehrfach durch den Wirkungsbereich der UV-Strahlungsquelle geleitet wird. Die erfindungsgemäße Vorrichtung bietet aufgrund ihrer speziellen Konstruktionsweise darüber hinaus weitere Möglichkeit zur Verlängerung der Verweilzeit, auf die weiter unten noch näher eingegangen werden wird.

Aufgrund der erhöhten Verweilzeit des zu behandelnden Mediums im Strahlungsbereich der UV-Strahlungsquelle besitzt die erfindungsgemäße Vorrichtung gegenüber vorbekannten Lösungen einen erheblich verbesserten Entkeimungs- und/oder Oxidationswirkungsgrad. In dem Medium enthaltene Mikroorganismen somit zuverlässig deaktivierbar. Und etwaige organische Schadstoffe lassen sich vollständig zu ungiftigen Verbindungen oxidieren bzw. abbauen.

Darüber hinaus ist es jedoch ein besonderer Vorteil der erfindungsgemäßen Vorrichtung, dass sich mit dieser innerhalb eines einzelnen Gerätes ein kombinierter UV-Entkeimungs- und UV-Oxidationsprozess (nachfolgend kurz kombinierter UV-Prozess genannt) durchführen lässt. Diese Möglichkeit ergibt sich primär durch die spezielle Schichtanordnung der aufeinanderfolgenden, von der UV-Strahlung durchstrahlbaren Behandlungskammern in Verbindung mit der Erhöhung der Verweilzeit des Medium und der Einwirkdauer der UV-Strahlung auf das Medium und dessen Verunreinigungen. Bei geeigneter Wahl des Spektralbereichs der UV-Strahlungsquelle wird bei dem kombinierten UV-Prozess das Medium sowohl durch Schädigung der DNS der enthaltenen Mikroorganismen entkeimt, als auch die molekulare Zusammensetzung der verbleibenden inaktivierten Mikroorganismen oder Viren durch photochemische Oxidation (z.B. im Rahmen einer Nassverbrennung) zerstört und vollständig abgebaut. Es ergibt sich somit ein sehr hoher Reinigungsgrad.

Bei der konventionellen UV-Entkeimung ggf. auftretende Unverträglichkeiten gegen bestimmte Proteine oder chemische Zusammensetzungen von inaktivierten aber physikalisch noch im Medium existenten Mikroorganismen oder -resten sind somit wirkungsvoll vermeidbar. Je nach Art der verwendeten UV-Strahlungsquelle bzw. der Anordnung ihrer UV-Einzelstrahlungsquelle kann bei dem so erzielbaren kombinierten UV-Prozess die UV-Entkeimung und UV-Oxidation gewissermaßen fließend ineinander über gehen oder aber auch sequentiell in aufeinanderfolgenden Behandlungskammern ausgeführt werden.

Mit der erfindungsgemäßen Vorrichtung ist somit ein Verfahren ausführbar, welches einem natürlichen, durch die UV-Strahlung der Sonne erzielbaren Prozess gleicht, bei dem letztendlich ungiftige, nicht mehr aktive Stoffe bzw. Verbindungen zurückbleiben, die ihrerseits wieder in den Biokreislauf zurückgeführt werden können.

Obwohl bei dem mit der erfindungsgemäßen Vorrichtung realisierbaren kombinierten UV-Prozess grundsätzlich separate Oxidationsmittel bei der UV-Oxidation eingesetzt werden könnten, ist dies aufgrund des erreichbaren hohen Wirkungsgrades in der Regel nicht erforderlich. Eine Gefährdung durch die Nutzung derartiger Chemikalien ist damit vermeidbar. Infolge der optimalen Ausnutzung der UV-Strahlung benötigt die erfindungsgemäße Vorrichtung auch nur eine UV-Strahlungsquelle mit einer vergleichsweise geringen Leistung. Die erfindungsgemäße Vorrichtung arbeitet ergo mit hohem Wirkungsgrad, niedrigen Betriebskosten, geringem Wartungsaufwand und folglich hoher Wirtschaftlichkeit.

Weitere bevorzugte und vorteilhafte Ausgestaltungsmerkmale der erfindungsgemäßen Vorrichtung sind Gegenstand der Unteransprüche 2 bis 35.

Die der Erfindung zugrunde liegende Aufgabe wird gemäß einem zweiten Aspekt gelöst durch die erfindungsgemäße Verwendung nach Anspruch 36. Diese sieht eine Verwendung der Vorrichtung nach einem oder mehreren der Ansprüche 1 bis 35 zur kombinierten UV-Entkeimung und UV-Oxidation (oben kombinierter UV-Prozess genannt), insbesondere zur Wasseraufbereitung vor.

Durch die erfindungsgemäße Verwendung sind im Wesentlichen die gleichen Vorteile zur erreichen, die zuvor im Zusammenhang mit der erfindungsgemäßen Vorrichtung dargelegt wurden.

Bevorzugte Ausführungsbeispiele der Erfindung mit zusätzlichen Ausgestaltungsdetails und weiteren Vorteilen sind nachfolgend unter Bezugnahme auf die beigefügten Zeichnungen näher beschrieben und erläutert.

### KURZE BESCHREIBUNG DER ZEICHNUNGEN

Es zeigt:
- Fig. 1: ein schematisches Blockschaltbild einer erfindungsgemäßen Vorrichtung gemäß einer ersten Ausführungsform;;
- Fig. 2: eine schematische Längsschnittansicht durch eine wesentliche Komponenten der erfindungsgemäßen Vorrichtung gemäß der ersten Ausführungsform;
- Fig. 3: eine schematische Querschnittsansicht entlang der Linie A-A in Fig. 2;
- Fig. 4: eine schematische Querschnittsansicht entlang der Linie B-B in Fig. 2;
- Fig. 5: eine schematische Querschnittsansicht entlang der Linie C-C in Fig. 2;
- Fig. 6: eine schematische Draufsicht auf die Vorrichtung von Fig. 2 mit Blickrichtung gemäß dem Pfeil D in Fig. 1;
- Fig. 7: eine schematische Längsschnittansicht durch eine erfindungsgemäßen Vorrichtung gemäß einer zweiten Ausführungsform;
- Fig. 8: eine schematische Querschnittansicht durch eine erfindungsgemäßen Vorrichtung gemäß einer dritten Ausführungsform;
- Fig. 9: eine schematische Querschnittansicht durch eine erfindungsgemäßen Vorrichtung gemäß einer vierten Ausführungsform;
- Fig. 10: eine schematische Längsschnittansicht durch einen wesentlichen Bereich einer erfindungsgemäßen Vorrichtung gemäß einer fünften Ausführungsform;
- Fig. 11: eine Vergrößerung des Bereiches X aus Fig. 10;
- Fig. 12: eine schematische Querschnittansicht durch einen wesentlichen Bereich einer erfindungsgemäßen Vorrichtung gemäß einer sechsten Ausführungsform;
- Fig. 13: eine schematische Längsschnittansicht durch eine erfindungsgemäße Vorrichtung gemäß einer siebten Ausführungsform;
- Fig. 14: eine schematische Querschnittsansicht entlang der Linie XIV-XIV in Fig. 13; und
- Fig. 15: eine schematische Querschnittsansicht entlang der Linie XV-XV in Fig. 13.

### DARSTELLUNG VON BEVORZUGTEN AUSFÜHRUNGSBEISPIELEN

In der nachfolgenden Beschreibung und in den Figuren werden zur Vermeidung von Wiederholungen gleiche Bauteile und Komponenten auch mit gleichen Bezugszeichen gekennzeichnet, sofern keine weitere Differenzierung erforderlich oder sinnvoll ist.

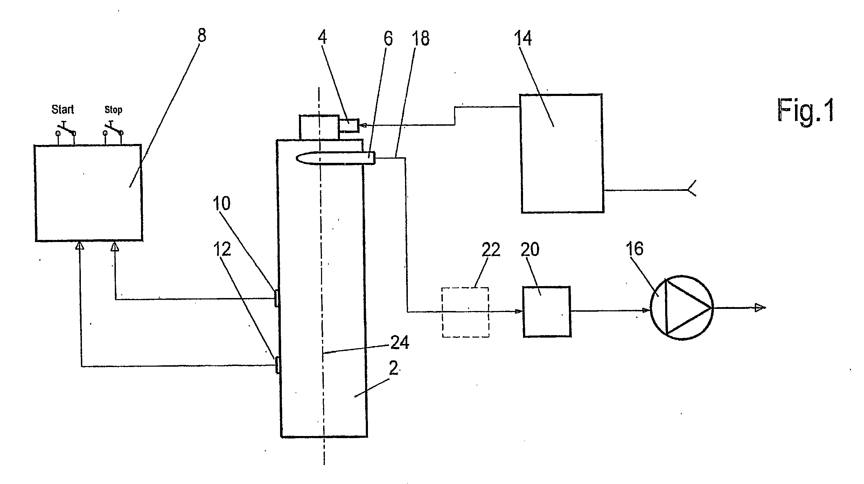

Fig. 1 zeigt ein schematisches Blockschaltbild einer erfindungsgemäßen Vorrichtung zur Behandlung eines flüssigen oder gasförmigen Mediums mittels UV-Strahlen, gemäß einer ersten Ausführungsform. Die Vorrichtung umfasst als wesentliche Komponenten einen fotochemischen Reaktor 2 mit einem Einspeisungskanal 4 für das zu behandelnde flüssige oder gasförmige Medium und einem Auslasskanal 6 für das behandelte Medium. Als zu behandelndes Medium wird im vorliegenden Ausführungsbeispiel Wasser verwendet, das durch Mikroorganismen bzw. Viren und organische Schadstoffe verunreinigt ist.

Der Reaktor 2 ist mit einer Kontrolleinrichtung 8 gekoppelt, die hier über zwei Sicherheits- und Kontrollsensoren 10, 12 (hier: ein optischer Sensor und ein Temperatursensor) verfügt. Die Vorrichtung besitzt des weiteren eine Vorfiltereinrichtung 14, die dem Einspeisungskanal 4 vorgeschaltet ist, sowie eine Pumpe 16, die in einer an den Auslasskanal 6 angeschlossenen Leitung 18 angeordnet ist. Zwischen dem Auslasskanal 6 und der Pumpe 16 ist ein Magnetventil 20 vorgesehen. In der Leitung 18 kann auch eine dem Auslasskanal 6 nachgeschaltete Filtereinrichtung 22 (z.B. ein Aktivkohlefilter) vorgesehen werden, welche zum Herausfiltem etwaiger Radikalenreste und/oder Ozonreste in dem behandelten Medium dient.

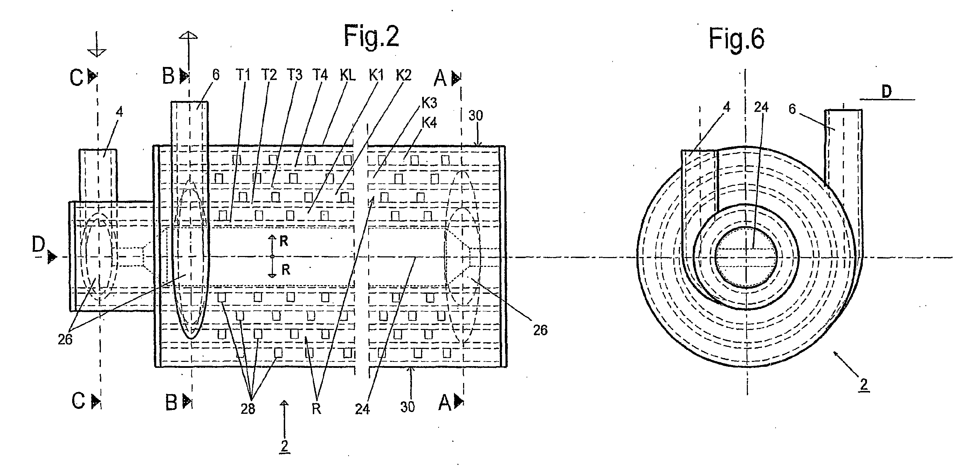

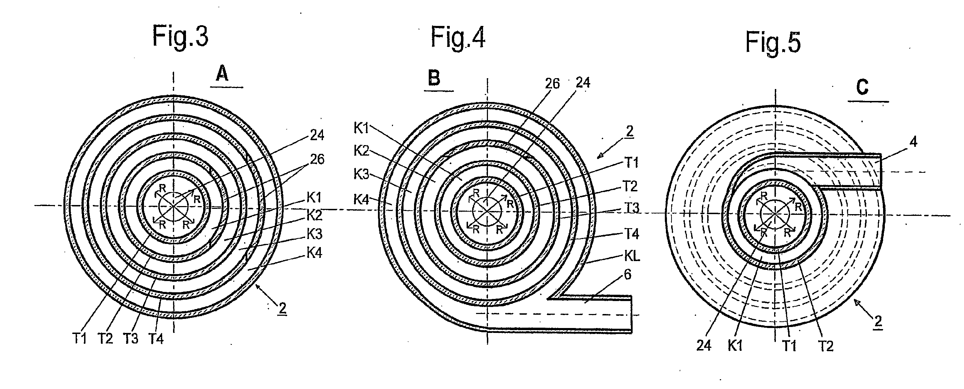

In Fig. 2 ist ein schematischer Längsschnittansicht durch den fotochemischen Durchflussreaktor 2 der erfindungsgemäßen Vorrichtung gemäß der ersten Ausführungsform dargestellt. Fig. 3 zeigt eine schematische Querschnittsansicht entlang der Linie A-A in Fig. 2. Die Vorrichtung bzw. der Reaktor 2 umfasst eine UV-Strahlungsquelle 24 mit einer axialen Längserstreckung und einer dazu im Wesentlichen senkrechten, insbesondere radialen Abstrahlungsrichtung R. Die UV-Strahlungsquelle 24 besitzt ein UV-Strahlungsspektrum von mindestens 180 nm bis zumindest 254 nm Wellenlänge. Im vorliegenden Fall liegt das UV-Strahlungsspektrum in einem Bereich von ca. 180nm bis ca. 260 nm.

Des weiteren besitzt die Vorrichtung mehrere Schichten von in Abstrahlungsrichtung übereinander angeordneten bzw. geschichteten, aufeinanderfolgenden, durchstrahlbaren Behandlungskammern K1 bis K4. Die Behandlungskammern K1 - K4 sind von der UV-Strahlungsquelle 24 und voneinander jeweils durch eine transparente, UV-durchlässige Trennschicht T1 bis T4 getrennt. Beginnend bei einer der UV-Strahlungsquelle 24 in Abstrahlungsrichtung R nächstliegenden, ersten Behandlungskammer K1, bilden die Behandlungskammem K1 - K4 jeweils einen entlang der Längserstreckung der UV-Strahlungsquelle 24 verlaufenden Durchströmungskanal für das Medium, der in die jeweils nachfolgende, von der UV-Strahlungsquelle 24 in Abstrahlungsrichtung R weiter entfernte Behandlungskammer mündet.

Wie in den Fig. 2 und 3 angedeutet ist, besitzt der Reaktor 2 im vorliegenden Beispiel vier Behandlungskammem K1, K2, K3, K4, deren Länge im Wesentlichen der axialen Länge der UV-Strahlungsquelle 24 entspricht. Die Behandlungskammern K1 - K4 sind in radialer Richtung konzentrisch um die UV-Strahlungsquelle 24 herum angeordnet. Der Querschnitt der Behandlungskammem K1 - K4 ist jeweils kreisringförmig. Die Behandlungskammern K5 - K5 sind durch fünf Trennschichten voneinander separiert. Zumindest die ausgehend von der UV-Strahlungsquelle 24 ersten vier Trennschichten T1 - T4 sind transparent und für UV-Strahlung durchlässig.

Bei der ersten Ausführungsform sind ausgehend von der UV-Strahlungsquelle 24 in Abstrahlungsrichtung R sowohl die erste Trennschicht T1, die zwischen der UV-Strahlungsquelle 24 und der ersten Behandlungskammer K1 angeordnet ist und der UV-Strahlungsquelle 24 am nächsten liegt, als auch die in radialer Richtung nachfolgende zweite Trennschicht T2 aus einem ersten Trennschicht-Material hergestellt. Dieses erste Trennschicht-Material besitzt eine UV-Durchlässigkeit, die im Wesentlichen das gesamte UV-Strahlungsspektrum der UV-Strahlungsquelle 24 einschließlich eines ersten Teilspektrums mit Wellenlängen kleiner 200nm, also auch die UV-Strahlung mit ca. 180 nm durchlässt. Die ersten beiden Trennschichten T1, T2 sind in Form von Glasrohren ausgestaltet, die aus synthetischem Quarzglas als das erste Trennschicht-Material hergestellt sind.

Grundsätzlich könnte die erste Trennschicht T1 bzw. das erste Glasrohr auch eine transparente Hüllenwandung der UV-Strahlungsquelle 24 selbst sein, die für den genannten Spektralbereich durchlässig ist. Dies bietet sich z.B. dann an, wenn als UV-Strahlungsquelle 24 eine in der ersten Behandlungskammer K1 angeordnete UV-Tauchlampe verwendet wird.

Die in Abstrahlungsrichtung R gemessene Gesamtdicke der Teilschicht, die aus den ersten beiden Glasrohren T1, T2 und den ersten beiden Behandlungskammern K1, K2 gebildet wird, in welche die UV-Strahlung durch das synthetische Quarzglas eintritt, ist so gewählt, dass sie im Wesentlichen einer maximalen effektiven Eindringtiefe des ersten Teilspektrums der UV-Strahlung entspricht, die sich in Abhängigkeit der Absorptionsfähigkeit des zu behandelnden Wassers und der UV-Durchlässigkeit der ersten beiden Glasrohre T1, T2 für dieses erste Teilspektrum ergibt. Die gewährleistet eine optimale Ausnutzung der kurzwelligen, harten UV-Strahlung kleiner 200 nm zum Zwecke einer nachfolgend noch beschriebenen Ozon- bzw. Radikalenerzeugung. Eine größere Schichtdicke ist nicht sinnvoll, sondern ggf. sogar gefährlich, da dann Bereiche des zu behandelnden Wassers in Abstrahlrichtung R nicht mehr zuverlässig durchdrungen bzw. erfasst werden könnten.

Die radial weiter außen liegenden Trennschichten, d.h. hier zumindest die dritte und vierte Trennschicht T3, T4 in Abstrahlungsrichtung R der ersten und zweiten Trennschicht T1, T2 nachfolgen, sind aus einem zweiten Trennschicht-Material hergestellt. Dieses zweite Trennschicht-Material besitzt eine UV-Durchlässigkeit, die von dem UV-Strahlungsspektrum der UV-Strahlungsquelle 24 nur UV-Strahlung in einem zweiten Teilspektrum mit Wellenlängen größer gleich 200nm durchlässt. Die dritte und vierte Trennschicht T3, T4 sind ebenfalls in Form von Glasrohren ausgebildet, die natürlichem Quarzglas als das zweite Trennschicht-Material hergestellt sind.

Die erfindungsgemäße Vorrichtung verfügt des Weiteren über eine Verweilzeit-Verlängerungseinrichtung zum Verlängern der Verweilzeit des zu behandelnden Wassers und der darin enthaltenen Verunreinigungen bzw. Schadstoffe im Strahlungsbereich der UV-Strahlungsquelle 24. Die aufeinanderfolgenden Behandlungskammern K1 - K4 bilden hierbei einen Teil der Verweilzeit-Verlängerungseinrichtung. Denn das Wasser strömt im Betrieb der Vorrichtung zunächst durch den Einspeisungskanal 4 in die erste, radial innere Kammer K1, in dieser entlang der axialen Längserstreckung der UV-Strahlungsquelle 24 bis zu einer endseitigen Austrittsöffnung 26, die gleichzeitig eine endseitige Eintrittsöffnung 26 für die nächste, d.h. die zweite, radial nachfolgende Behandlungskammer K2 bildet. Hierbei wird das Wasser um ca. 180° umgelenkt, strömt wieder entlang der Längserstreckung der UV-Strahlungsquelle 24 und tritt in die dritte, radial weiter außen liegende Behandlungskammer K4, wobei es wiederum um 180° umgelenkt wird. Von der dritten Behandlungskammer K3 gelangt es auf analoge Art und Weise in die vierte, radial äußerste Kammer K4 und verlässt die Vorrichtung durch den Auslasskanal 6. Auf dem gesamten Strömungsweg durch die Behandlungskammern K1 - K4 ist das Wasser der UV-Strahlung ausgesetzt.

Die Verweilzeit-Verlängerungseinrichtung umfasst jedoch noch weitere Detailelemente, welche nachfolgend unter Bezugnahme auf die Fig. 4 bis 6 beschrieben werden. Hierbei zeigt Fig. 4 eine schematische Querschnittsansicht entlang der Linie B-B in Fig. 2; Fig. 5 eine schematische Querschnittsansicht entlang der Linie C-C in Fig. 2; und Fig. 6 eine schematische Draufsicht auf die Vorrichtung von Fig. 2 mit Blickrichtung gemäß dem Pfeil D in Fig. 1.

Wie in der Fig. 4 angedeutet ist, ist der Einspeisungskanal 4 als Teil der Verweilzeit-Verlängerungseinrichtung so ausgestaltet, dass er bezogen auf die radiale Abstrahlungsrichtung R bzw. die kreisringförmige Querschnittsform der ersten Behandlungskammern K1 im Wesentlichen tangential in die erste Behandlungskammer K1 mündet. Dem einzuspeisenden Wasser wird dadurch ein Drall verliehen; so dass es in der ersten Behandlungskammer K1 auf einer annähernd spiralförmigen Bahn um die UV-Strahlungsquelle 24 herum von der Einspeisungsöffnung 4 zu der Austrittsöffnung 26 der ersten Behandlungskammer K1 strömt. Die Austrittsöffnung 26 der ersten Behandlungskammer K1 bzw. die Eintrittsöffnung 26 der zweiten Behandlungskammer K2 ist z.B. schlitzförmig mit einem linsenartigen Schlitzquerschnitt ausgestaltet (vgl. auch Fig. 2 und 3), so dass das Wasser weitgehend ungehindert in Drallrichtung in die zweite Behandlungskammer K2 strömen kann. Dieser Vorgang wird durch die infolge des Dralls hervorgerufenen Fliehkräfte unterstützt. Die folgenden Austritts- bzw. Eintrittsöffnung 26 der jeweiligen radial nachfolgenden Behandlungskammern K3, K4 sind entsprechend konstruiert.

Auch der Auslasskanal 6 ist als Teil der Verweilzeit-Verlängerungseinrichtung ausgestaltet. Bezogen auf die radiale Abstrahlungsrichtung R bzw. die kreisringförmige Querschnittsform der Behandlungskammern K1 - K4 tritt er im Wesentlichen tangential und in Drallrichtung aus der letzten, der UV-Strahlungsquelle 24 in Abstrahlungsrichtung R am weitesten entfernten Behandlungskammer K4 heraus und trägt somit zur Aufrechterhaltung der spiralförmigen Strömung um die UV-Strahlungsquelle 24 bei.

Zusätzlich kann die Verweilzeit-Verlängerungseinrichtung Verwirbelungselemente 28 oder Turbulatoren aufweisen, die zum Beispiel in den Behandlungskammern K1 - K4, dem Einspeisungskanal 4, dem Auslasskanal 6, Eintritts- oder Austrittsöffnungen 26 zwischen aufeinanderfolgenden Behandlungskammern oder weiteren Zu- und Ableitungen angeordnet sind. Als Verwirbelungselemente 28 kommen z.B. noppenartige Erhöhungen, Vertiefungen, Schaufeln, Flügel, Zäune, rotierende oder schwingende Teile o.ä. auf den Wandungen der genannten Komponenten in Betracht. Sofern diese Verwirbelungselemente 28 in Verbindung mit dem besagten tangentialen Einspeisungskanal 4 und Auslasskanal 6 verwendet werden, sollten sie zur Aufrechterhaltung der grundlegenden Drallrichtung beitragen. Die Verwirbelungselemente 28 können fest installiert oder aber auch an herausnehmbaren Einsätzen angeordnet sein, was Reinigungsarbeiten erleichtert. In der Fig. 2 sind Wirbel-Noppen angedeutet, die an den Glasrohren T1 - T4 angeordnet sind.

Die letzte, d.h. hier die vierte Behandlungskammer K4 besitzt an ihrer von der UV-Strahlungsquelle 24 in Abstrahlungsrichtung am weitesten entfernten Seite eine UV-Reflektionseinrichtung 30, welche die von der UV-Strahlungsquelle 24 emittierte und bis zur äußeren Wandung KL durchgedrungene UV-Strahlung wieder zurückreflektiert und somit zu einem hohen Ausnutzungsgrad der UV-Strahlung und einer intensiven Behandlung des Wassers beiträgt. Falls die äußere Wandung KL der letzten Behandlungskammer K4 für das UV-Licht undurchlässig ist, kann es sich bei der UV-Reflektionseinrichtung 30 z.B. um eine auf die Innenseite dieser Wandung KL aufgetragende Spiegelschicht oder eine stark polierte, reflektierende Oberfläche handeln. Ist die äußere Wandung KL für die UV-Strahlung hingegen durchlässig (z.B. aus natürlichem Quarzglas, so kann die UV-Reflektionseinrichtung 30 z.B. in Form einer um das Glas herum angebrachten Reflektions- oder Spiegelschicht oder dergleichen ausgeführt werden.

Es wird nun die Funktionsweise der erfindungsgemäßen Vorrichtung beschreiben werden.

Die Vorrichtung wird über die Kontrolleinrichtung 8 in Betrieb gesetzt und die UV-Strahlungsquelle 24 aktiviert. Nach einer vorgegebenen Einbrennzeit wird das Magnetventil 20 geöffnet und die Pumpe 16 betätigt. Kontaminiertes Wasser wird über die Vorfiltereinrichtung 14 in den Reaktor 2 und durch dessen Behandlungskammern K1 - K4 hindurch gesaugt, dort entkeimt und von chemischen Verunreinigungen befreit und fließt über den Auslasskanal 6, den eventuellen Nachfilter 22, das Magnetventil 20 und die Pumpe 16 aus.

Der optische Sensor 10 hat im Betrieb die Aufgabe, eine Trübung der Glaswände der Glasrohre T1 - T4 zu erfassen. Eine Trübung kann z.B. durch Kalkablagerungen und/oder durch trübes Wasser verursacht werden. Sie würde die Leistung des Reaktors einschränken. Bei einer definierten Trübung wird dies der Kontrolleinrichtung 8 über ein entsprechendes Sensorsignal gemeldet. Bei einer zu starken Trübung schaltet die Kontrolleinrichtung 8 die UV-Strahlungsquelle 24, das Magnetventil 20 und die Pumpe 16 ab und lässt einen erneuten Start nicht zu. Erst nach einer Reinigung oder Fehlerbeseitigung wird der Betrieb wieder freigegeben. Der Temperatursensor 12 hat die Aufgabe, den Reaktor vor Überhitzung zu schützen. Bei einem eventuell blockiertem Wasserfluss, würde sich der Reaktor aufheizen und das darin befindliche Wasser eventuell kochen. Der Sensor 12 meldet solch einen gefährlichen Zustand an die Kontrolleinrichtung 8 und diese schaltet dann die UV-Strahlungsquelle 24, das Magnetventil 20 und die Pumpe 16 ab.

Der Reinigungsvorgang als solcher läuft in de erfindungsgemäßen Vorrichtung wie folgt ab:

Das schadstoffbelastete, mit Mikroorganismen bzw. Viren kontaminierte Wasser wird in der ersten und zweiten Behandlungskammer K1, K2 mit UV-Strahlung beaufschlagt, welches ein UV-Strahlungsspektrum mit Wellenlängen von sowohl kleiner als auch größer 200nm besitzt. Die UV-Strahlung größer 200 nm, insbesondere um 254 nm, bewirkt hierbei die UV-Entkeimung durch Zerstörung der DNS der im Wasser enthaltenen Mikroorganismen. Die Strahlung unterhalb 200nm, insbesondere um ca. 180 nm setzt die UV-Oxidation im Rahmen einer photochemischen Nassverbrennung in Gang.

Die Strahlung unter 200nm produziert aus dem im Wasser befindlichen molekularen Sauerstoff O₂) Ozon (O₃). Dies erfolgt in der ersten und zweiten Kammer. Das Ozon wird durch die vorhandene UV-Strahlung oberhalb von 200nm in einzelne (single) Sauerstoffatome (O) zerlegt. Diese an sich schon hoch reaktiven Sauerstoffatome verbinden sich infolge der fotochemischen Reaktion mit Wasserstoffatomen (H+) zu OH-Molekülen. OH-Moleküle sind freie Radikale, welche im Wesentlichen alle Arten der zuvor beschriebenen organischen Schadstoffe, insbesondere die molekularen Reste oder Hüllen der deaktivierten Mikroorganismen, zerstört. OH-Radikale besitzen eine max. Lebensdauer von ca. 100 Millisekunden. Danach zerfallen sie wieder zu Wasserstoff und Sauerstoff und damit zu absolut ungiftigen Stoffen. Es bleiben bei einer photochemischen Reaktion keinerlei schädliche Substanzen zurück. Alle organischen Stoffe werden völlig oxidiert (verbrannt). Auch Stoffe wie z.B. Pflanzenschutzmittel (DDT, Atrazine usw.) oder Zwischenprodukte von chemischen Reaktionen werden zu ungiftigen Resten oxidiert.

Bei der erfindungsgemäßen Vorrichtung erfolgt die Radikalerzeugung in den ersten beiden Behandlungskammern K1, K2. Da das die Radikale enthaltene Wasser fortwährend weiterströmt, findet die Oxidationen ("der Verbrauch") der Radikale hingegen weitgehend in der dritten und vierten Behandlungskammer K3, K4 statt. Die oben beschriebene inhärente bzw. zusätzlich ausgestaltete Verweilzeit-Verlängerungsvorrichtung der erfindungsgemäßen Vorrichtung ermöglicht die Erzeugung einer hohen Radikalendichte und steigert durch die Verwirbelung des Wassers gleichzeitig die Treffsicherheit der OH-Radikale auf die zu zerstörenden Molekularstrukturen. Die zur photochemischen Nassverbrennung notwendige Verweilzeit wird mit der erfindungsgemäßen Konstruktion mehr als erfüllt. Die notwendige Verweilzeit zur Oxidation von Eiweißkörpern liegt z.B. bei ca. 100 mS. Die bei einer Strömungsgeschwindigkeit von z.B. 3,8l/min in dem Reaktor der erfindungsgemäßen Vorrichtung erzielbare max. Verweilzeit liegt bei ca. 6000 - 7000 mS.

Da das dritte und vierte Glasrohr T3, T4 aus normalem bzw. natürlichem Quarzglas hergestellt ist und UV-Strahlung unterhalb 200nm nicht durchlässt und die UV-Strahlung unterhalb 200nm zudem bereits in der ersten und zweiten Behandlungskammer K1, K2 im Wesentlichen vollständig absorbiert wurde, ist sie in der dritten und vierten Behandlungskammer K3, K4 nicht mehr wirksam. Für die dritte und vierte Behandlungskammer K3, K4 sind die Trennschichten T3, T4 aus normalem bzw. natürlichem Quarz, welches für UV-Strahlung oberhalb von 200nm durchlässig ist, daher völlig ausreichend. Aus der letzten Behandlungskammer K4 tritt das Wasser tangential aus und steht dann entkeimt und von organischen Schadstoffen und Rückständen von Mikroorganismen bzw. Viren befreit zur Verfügung.

In der erfindungsgemäßen Vorrichtung findet somit fortlaufend eine kombinierte UV-Entkeimung und UV-Oxidation statt (weiter oben als kombinierter UV-Prozess bezeichnet), welcher einen sehr hohen Reinigungsgrad des kontaminierten Wassers gewährleistet.

Nach der Behandlung im Reaktor 2 sollte das entkeimte und gereinigte Wasser zur Sicherheit über Aktivkohle geleitet werden, was in der Nachfiltereinrichtung 22 erfolgen kann. Aktivkohle stellt wieder einen natürlichen Redox-Wert her. Denn bedingt durch die OH-Radikale herrscht in den Behandlungskammern K1 - K4 des Reaktors 2 ein enorm hohes Redoxpotential von bis zu 2Volt. Dieses ist schädlich und muss wieder auf die natürlichen Verhältnisse (250 - 450 mV) eingestellt werden.

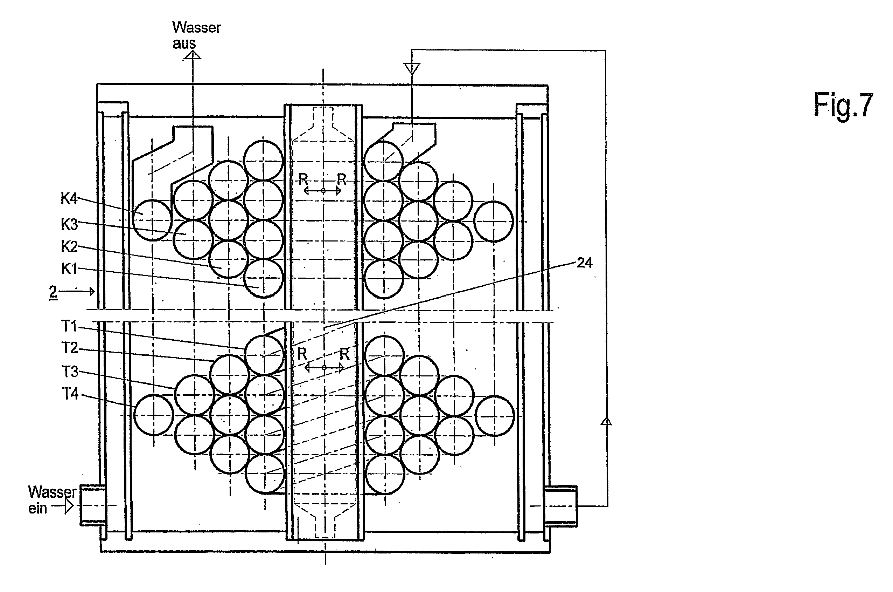

Fig. 7 zeigt eine schematische Längsschnittansicht durch eine erfindungsgemäßen Vorrichtung gemäß einer zweiten Ausführungsform. Bei dieser Variante erstrecken sich die jeweiligen, schichtweise angeordneten Behandlungskammern K1 - K4 jeweils spiralförmig um die UV-Strahlungsquelle 24 herum.

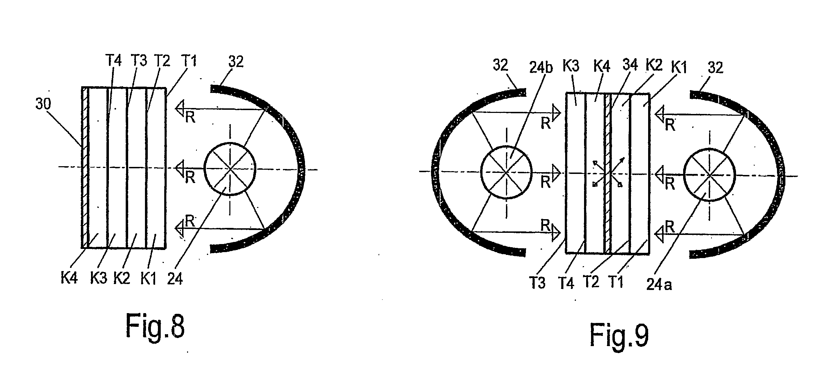

Fig. 8 zeigt eine schematische Querschnittansicht durch eine erfindungsgemäße Vorrichtung gemäß einer dritten Ausführungsform. Das Funktionsprinzip dieser Variante entspricht im Wesentlichen dem der ersten Ausführungsform, jedoch sind die schichtweise angeordneten Behandlungskammern K1 - K4 in einer plattenartigen bzw. kastenartigen Anordnung gruppiert, und die UV-Strahlungsquelle 24 ist seitlich dieser Anordnung platziert. Jede Behandlungskammer K1 - K4 besitzt eine meanderförmige Strömungsbahn (nicht gezeigt) für das zu behandelnde Medium. Für eine höhere UV-Lichtausbeute ist die Vorrichtung mit einer parabolischen Reflektoreinrichtung 32 ausgestattet, welche das von der UV-Strahlungsquelle 24 emittierte UV-Licht auf die geschichteten Behandlungskammern K1 - K4 bündelt.

In der Fig. 9 ist eine schematische Querschnittansicht durch eine erfindungsgemäßen Vorrichtung gemäß einer vierten Ausführungsform dargestellt. Die Ausführungsform nach Fig. 9 ähnelt vom Prinzip her der nach Fig. 8, jedoch sind zwei UV-Einzelstrahlungsquellen 24a, 24b vorgesehen, welche die geschichteten Behandlungskammern K1 - K4 von zwei unterschiedlichen Seiten her durchstrahlen. Die erste UV-Einzelstrahlungsquelle 24a emittiert hierbei UV-Licht in einem Spektrum kleiner 200 nm und die zweite 24b in einem Spektrum größer 200 nm. Wird diesen UV-Einzelstrahlungsquelle 24a, 24b jeweils eine Gruppe von Behandlungskammern zugeordnet und zwischen diesen z.B. jeweils ein Filter oder eine trennende UV-Reflektionseinrichtung 34 angeordnet, so kann innerhalb der Vorrichtung die UV-Entkeimung und UV-Oxidation zeitlich und örtlich getrennt ausgeführt werden, z.B. erst nur die UV-Entkeimung und anschließend die UV-Oxidation. Als Filter können hierbei auch das erste oder zweite Material für die jeweiligen transparenten, UV-durchlässigen Trennschichten fungieren.

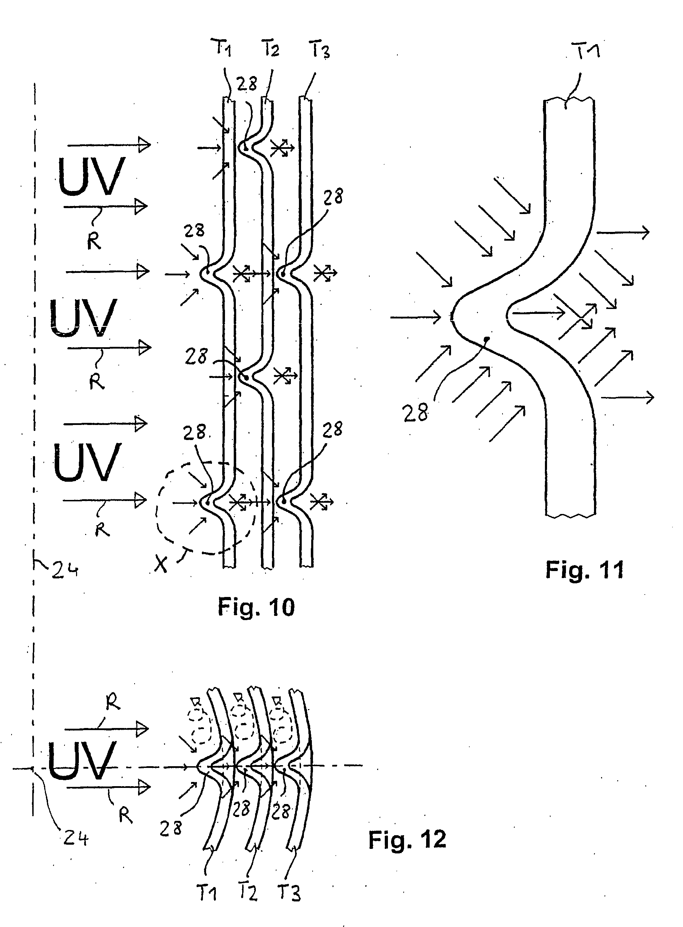

Fig. 10 zeigt eine schematische Längsschnittansicht durch einen wesentlichen Teilbereich einer erfindungsgemäßen Vorrichtung gemäß einer fünften Ausführungsform. Bei dieser Variante sind die Verwirbelungselemente 28 bzw. Wirbelnoppen der Verweilzeit-Verlängerungseinrichtung transparent und UV-durchlässig ausgebildet. Die transparenten und UV-durchlässigen Verwirbelungselemente 28 sind gleichzeitig integraler Bestandteil einer jeweiligen transparenten, UV-durchlässigen Trennschicht T1, T2, T3 .

Genauer gesagt bilden in diesem Beispiel dreidimensional verformte Bereiche der jeweiligen Trennschicht T1, T2, T3 die transparenten und UV-durchlässigen Verwirbelungselemente 28. Sie können zum Beispiel bei der Herstellung der die Trennschichten T1, T2, T3 bildenden Glasrohre durch plastisches Verformen der Glasrohre unter Wärmeeinwirkung hergestellt werden. Ebenso ist es jedoch möglich, separate, d.h. als unabhängige Einzelteile ausgestaltete transparente und UV-durchlässige Verwirbelungselemente 28 zu verwenden, die an oder auf der betreffenden transparenten, UV-durchlässigen Trennschicht T1, T2, T3 befestigt werden.

Im vorliegenden Fall sind die transparenten, UV-durchlässigen Verwirbelungselemente 28 als optische Linsen (z.B. Sammellinsen oder Streulinsen) und/oder als Lichtleiter mit genau definierten optischen Eigenschaften ausgebildet. Sie besitzen also eine Doppelfunktion, denn sie verwirbeln zum einen das zu behandelnde Medium und erhöhen dadurch dessen Verweilzeit in der UV-Strahlung und zum anderen fokussieren (bzw. streuen) sie die UV-Strahlen in einer genau vorbestimmten Art und Weise und können dadurch eine erhöhte Ausbeute der UV-Strahlung erzielen. Sie werden deshalb nachfolgend kurz opto-mechanische Noppen 28 genannt. Bei Betrachtung in Abstrahlungsrichtung R besitzen die opto-mechanischen Noppen 28 eine im Wesentlichen kreisrunde Grundrissform. Sie können je nach Ausgestaltungsform jedoch auch oval, viereckig, rechteckig, polygonal geformt sein oder eine unregelmäßige bzw. asymmetrische Grundrissform aufweisen.

Wie in der Fig. 10 erkennbar ist, wölben sich die opto-mechanischen Noppen 28 der jeweiligen Trennschichten T1, T2, T3 in diesem Beispiel mit ihrer Ausbuchtung entgegen der Abstrahlungsrichtung R der UV-Strahlen. Mit anderen Worten sind sie auf der der UV-Strahlung zugewandten Seite der jeweiligen Trennschicht T1, T2, T3 angeordnet. Je nach Linsentyp, den eine opto-mechanische Noppe 28 bildet, können die opto-mechanischen Noppen 28 jedoch auch auf der der UV-Strahlung abgewandten Seite oder sogar auf beiden Seiten angeordnet sein. Die Rückseite der Noppen 28 bildet eine Einbuchtung, die wie die Ausbuchtung auf der Vorderseite wiederum als ein Verwirbelungselement dient. Wie aus der Fig. 10 des Weiteren deutlich hervorgeht, sind die Noppen 28 einer ersten Trennschicht (z.B. T1) gegenüber den Noppen einer zweiten Trennschicht (z.B. T2) seitlich versetzt.

Die Noppenhöhe bzw. der Abstand der Noppenspitzen ist in Abhängigkeit der in Abstrahlungsrichtung R gemessenen Breite einer durch die jeweiligen Trennschichten T1, T2, T3 begrenzten Behandlungskammer so gewählt, dass die Höhe bzw. der Abstand einen vorbestimmten Wert aufweist. Genauer gesagt ist der Abstand einer optischen Eingangsebene (oder Ausgangsebene) eines opto-mechanischen Noppens 28 einer in Abstrahlungsrichtung R von der UV-Strahlungsquelle distal angeordneten Trennschicht (z.B. T2) in einem Abstand von der optischen Ausgangsebene (oder Eingangsebene) einer in Abstrahlungsrichtung R von der UV-Strahlungsquelle proximal angeordneten Trennschicht (z.B. T1) angeordnet, der vorzugsweise kleiner gleich 15, insbesondere kleiner gleich 10 mm ist (je nach Ausgestaltungsform insbesondere auch kleiner gleich 9 mm, kleiner gleich 8 mm, kleiner gleich 7 mm, kleiner gleich 6 mm, kleiner gleich 5 mm, kleiner gleich 4 mm, kleiner gleich 3 mm, kleiner gleich 2 mm, kleiner gleich 1 mm, kleiner gleich 0,5 mm, kleiner gleich 0,25 mm). So ist die Noppenspitze bzw. der Noppenkopf eines opto-mechanischen Noppens 28 der Trennschicht T2 in diesem Beispiel ca. 0,5 mm von der der UV-Strahlungsquelle abgewandten Seite der Trennschicht T1 beabstandet, so dass die o.g. Bedingung erfüllt ist. Für die Noppen 28 der weiteren Trennschichten (hier: T3 in Bezug zu T2) werden vorzugsweise entsprechende Abstände gewählt.

Auf diese Weise muss sich die UV-Strahlung mit einer Wellenlänge kleiner 200nm (hier: z.B. 185 nm) zwischen der jeweiligen optischen Ausgangs- und Eingangsebene nur eine kurze Strecke von max. ca. 10 mm durch das zu behandelnde Medium (z.B. Wasser) bewegen und wird nicht zu stark gedämpft. Durch die opto-mechanischen Noppen 28 kann diese kurzweilige UV-Strahlung daher in weiter von der UV-Strahlungsquelle 24 angeordnete Behandlungskammern transportiert werden und dort zur Photoreaktion/UV-Oxidation und zur Bildung von Ozon bzw. Radikalen verwendet werden.

Der gleiche Effekt ist mit Hilfe der opto-mechanischen Noppen 28 bei Bedarf auch für die längerwellige, entkeimend wirkende UV-Strahlung größer 200nm (z.B. 254 nm) realisierbar. Grundsätzlich sollte der jeweilige Abstand in Abhängigkeit der jeweiligen Wellenlänge des zu betrachtenden UV-Tellspektrums, der durch den gegenseitigen Abstand der Trennschichten bestimmten Breite der Behandlungskammer und damit der von der UV-Strahlung im zu behandelnden Medium zu überbrückenden Strecke zwischen zwei benachbarten Trennschichten und der Art des zu behandelnden Mediums gewählt werden.

Die erfindungsgemäße Vorrichtung kann somit ohne eine Erhöhung der Leistung bzw. des elektrischen Energieverbrauchs der UV-Strahlungsquelle mit einer größeren Anzahl von Behandlungskammern ausgestattet werden und eine größere Menge des zu behandelnden Mediums reinigen, als dies ohne die opto-mechanischen Noppen 28 möglich wäre. Der erreichbare Nutzungsgrad ist hierbei insbesondere von der Anzahl der opto-mechanischen Noppen 28, deren optischer Güte und dem zu behandelnden Medium abhängig.

In der Fig. 11, die eine Vergrößerung des Bereiches X aus Fig. 10 zeigt, ist der durch einen opto-mechanischen Noppen 28 erzeugte Strahlengang der UV-Strahlen nochmals deutlicher dargestellt. Wie zu erkennen ist, wirkt der opto-mechanische Noppen 28 im Wesentlichen als eine Sammellinse (in der Art einer Domlinse), welche die ankommenden UV-Strahlen fokussiert und gebündelt weiterleitet.

Fig. 12 zeigt eine schematische Querschnittansicht durch einen wesentlichen Bereich einer erfindungsgemäßen Vorrichtung gemäß einer sechsten Ausführungsform. Diese Variante ähnelt weitgehend der von Fig. 10 und 11. Jedoch sind in diesem Fall die opto-mechanischen Noppen 28 jeweils aufeinanderfolgender Trennschichten T1, T2, T3 in Abstrahlungsrichtung R der UV-Lichtquelle gesehen hintereinander angeordnet. Eine Mischform aus der Anordnungsweise der Noppen 28 in Fig. 10, 11 und 12 ist ebenfalls möglich. In der Fig. 12 ist durch dünne, gestrichelte Linien auch nochmals die durch die opto-mechanischen Noppen 28 verursachte Verwirbelung des zu behandelnden Mediums angedeutet, welche eine Erhöhung der Verweilzeit des Mediums in den UV-Strahlen bewirkt.

Bei der erfindungsgemäßen Vorrichtung nach Fig. 12 sind die in Abstrahlungsrichtung R der UV-Lichtquelle gesehen hintereinander angeordneten opto-mechanischen Noppen 28 so zueinander ausgerichtet, dass eine optische Ausgangsebene (bzw. Eingangsebene) einer opto-mechanischen Noppe 28 einer ersten Trennschicht (z.B. T1) in einem Abstand von einer optischen Eingangsebene (bzw. Ausgangsebene) einer opto-mechanischen Noppe 28 einer in Abstrahlungsrichtung R nachfolgenden, zweiten Trennschicht (z.B. T2) angeordnet ist, der vorzugsweise kleiner gleich 15mm, insbesondere kleiner gleich 10 mm ist (in bestimmen Anwendungsfällen insbesondere auch kleiner gleich 9 mm, kleiner gleich 8 mm, kleiner gleich 7 mm, kleiner gleich 6 mm, kleiner gleich 5 mm, kleiner gleich 4 mm, kleiner gleich 3 mm, kleiner gleich 2 mm, kleiner gleich 1 mm, kleiner gleich 0,5 mm, kleiner gleich 0,25 mm). Dadurch sind die gleichen Effekte und Vorteile zu erzielen, die bereits weiter oben in Zusammenhang mit den äquivalenten Abständen bei der Vorrichtung nach Fig. 10 beschrieben wurden.

In den Ausführungsformen nach Fig. 10 bis 12 können die transparenten, UV-durchlässigen Trennschichten T1, T2, T3 je nach Krümmung ebenfalls optische Bündelungs- oder Streueffekte bewirken, die mit denen der opto-mechanischen Noppen 28 kombinierbar sind.

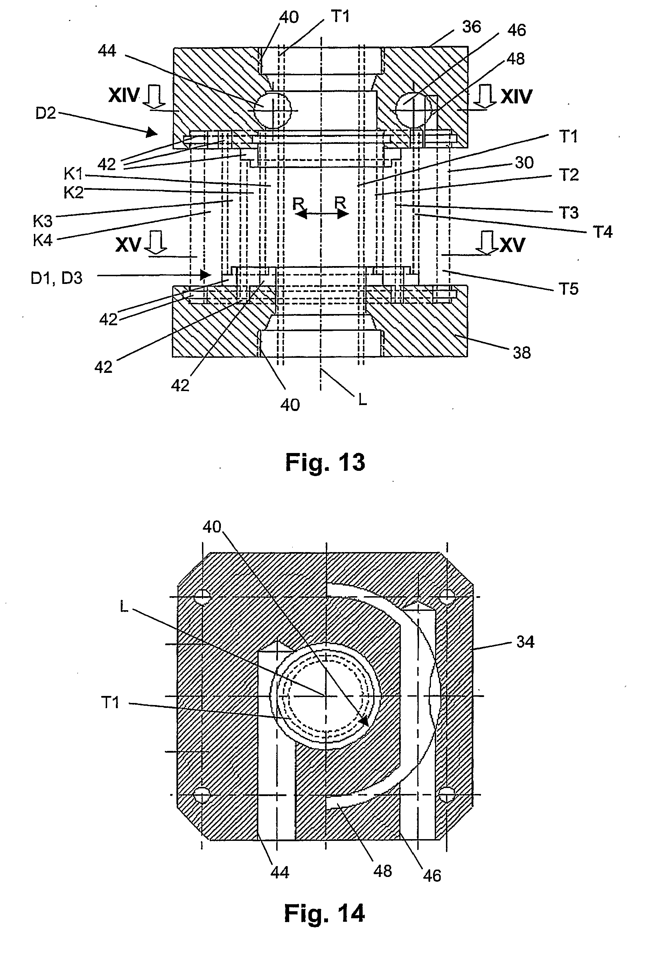

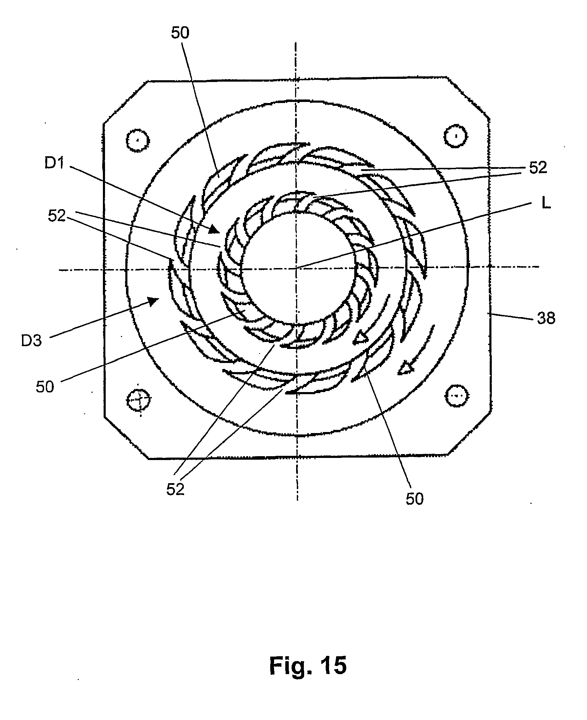

In der Fig. 13 ist eine schematische Längsschnittansicht durch eine erfindungsgemäße Vorrichtung gemäß einer siebten Ausführungsform dargestellt. Fig. 14 ist eine schematische Querschnittsansicht entlang der Linie XIV-XIV in Fig. 13. Und Fig. 15 eine schematische Querschnittsansicht entlang der Linie XV-XV in Fig. 13. Diese siebte Variante entspricht von ihrem Grundprinzip her den zuvor erläuterten Ausführungsformen, weist gegenüber diesen jedoch noch einige Besonderheiten auf.

So besitzt die Vorrichtung nach Fig. 13 einen oberen 36 und einen unteren demontierbaren Deckel 38, zwischen denen sich vier konzentrisch angeordnete runde Quarzglasrohre, welche die UV-durchlässigen Trennschichten T1 - T4 bilden, sowie ein äußeres rundes Quarzglasrohr T5 erstrecken, welches als eine UV-Reflektionseinrichtung 30 bildet. Das äußere Quarzglasrohr T5 ist an seiner Außenseite durch Aufdampfen einer Aluminiumschicht verspiegelt und reflektiert in Strahlungsrichtung R eintreffende und durch das Quarzglasrohr T5 bis zur Aluminiumschicht hindurchtretende UV-Strahlen entgegen der Strahlungsrichtung R wieder zurück. Alle diese Quarzglasrohre T1 - T5 sind gegenüber den Deckeln 36, 38 durch nicht gezeigte Dichtungen abgedichtet. Auf diese Weise ist eine einfache Austauschbarkeit der jeweiligen Komponenten und insbesondere eine simple Montage und Demontage der Quarzglasrohr T1- T5 sowie eine vereinfachte Wartung oder Reparatur realisierbar.

Das innerste Quarzglasrohr T1 erstreckt sich über jeweils eine zentrale Durchgangsöffnung 40 längs durch die Deckel 36, 38 hindurch und ist an mindestens einer Seite offen oder zu öffnen. Dadurch kann die UV-Strahlungsquelle (hier der besseren Übersichtlichkeit nicht gezeigt) durch die Rohröffnung hindurch im Inneren des Rohres T1 und der Vorrichtung angebracht oder bei Bedarf wieder entfernt werden, ohne dass eine Demontage der Trennschichten T1 - T5, oder der Deckel 36, 38 erforderlich ist.

Die Deckel 36, 38 verfügen jeweils über eine Zentrierungseinrichtung 42 für die dazwischen angeordneten UV-durchlässigen Trennschichten T1 - T5 bzw. Rohre, so dass diese einfach und sicher an einer vorbestimmten Position zueinander und an den Deckeln 36, 38 angeordnet werden können. Die Zentrierungseinrichtung 42 umfasst in diesem Beispiel Nuten und/oder Vorsprünge und/oder Absätze oder dergleichen, in welche bzw. auf oder an welche die jeweiligen Rohre T1 - T5 einsteckbar oder aufsteckbar sind. Das innerste Rohr T1 wird durch die Durchgangsöffnungen 40 sowie seine Dichtungen zentriert. Aufgrund dieser Konstruktion ist es nicht erforderlich, die Quarzglasrohre T1 - T5 in die Deckel 36, 38 einzugießen oder einzukleben, was eine Wartung oder Reparatur der Vorrichtung weiter erleichtert.

Wie in den Fig. 13 und 14 erkennbar ist, besitzt der obere Deckel 36 einen Einlass 44 und einen Auslass 46 für das zu behandelnde Medium. Grundsätzlich könnten Einlass 44 und Auslass 46 auch am unteren Deckel 38 vorgesehen sein. Ebenso ist es möglich, dass ein jeweiliger Deckel 36, 38 entweder nur Einlass 44 oder Auslass 46 aufweist. Der Einlass 44 mündet in die radial innerste Behandlungskammer K1. Der Auslass 46 kommuniziert über einen halbkreisförmigen Kanal 48 mit der radial äußersten Behandlungskammer K4.

Damit das zu behandelnde Medium von einer Behandlungskammer zur jeweils nächsten gelangen kann, sind im oberen Deckel 36 Verbindungskanäle 50 oder "Übergabekanäle" vorgesehen, welche eine Behandlungskammer K1 - K3 mit der jeweils nachfolgenden Behandlungskammer K2 - K4 verbinden (siehe z.B. Fig. 15). Die Verbindungskanäle 50 können grundsätzlich auch in dem unteren Deckel 38 oder in beiden Deckeln 36, 38 angeordnet sein.

Die siebte Ausführungsform verfügt zudem über eine weiter ausgestaltete Verweilzeit-Verlängerungseinrichtung. D.h., alternativ zu oder zusätzlich zu den oben beschrieben Komponenten der Verweilzeit-Verlängerungseinrichtung weist diese einen Teil auf, der hier in dem oberen 36 und unteren Deckel 36 angeordnet ist. Genauer gesagt, besitzt der im oberen 36 und unteren Deckel 38 angeordnete Teil der Verweilzeit-Verlängerungseinrichtung eine Verwirbelungseinrichtung für das zu behandelnde Medium. Diese Verwirbelungseinrichtung ist in diesem Ausführungsbeispiel in die Verbindungskanäle integriert.

Die Verwirbelungseinrichtung umfasst eine Düseneinrichtung mit einer Vielzahl von Düsen 50, deren Düsenkanäle als die Verbindungskanäle 52 ausgebildet sind, wie in der Fig. 15 angedeutet ist (in der Fig. 15 sind die Quarzglasrohre T1 - T5 der besseren Übersichtlichkeit halber nicht dargestellt).

Zwischen jeweils zwei aufeinanderfolgenden Behandlungskammern (hier: K1-K2, K2-K3, K3-K4) ist eine Vielzahl von Düsen 50 vorgesehen, welche bezogen auf eine Längsachse L der Behandlungskammern (K1 - K4) bzw. der Vorrichtung kranzartig um die Längsachse L herum angeordnet sind. Die Düsen 50 eines solchen Düsenkranzes werden nachfolgend kurz als Düsenstufen D1 bis D3 bezeichnet. In der Fig. 15 sind am unteren Deckel 38 eine erste und eine zweite Düsenstufe D1 und D3 erkennbar. Eine dritte Düsenstufe (D3) befindet sich am oberen Deckel 36. Zwischen dem Einlass 44 und der ersten Behandlungskammer K1 ist in diesem Beispiel keine Düsenstufe notwendig, da der Einlass 44 im Wesentlichen tangential in die erste Behandlungskammer K1 mündet, so dass das einströmende Medium in der Kammer K1 um die UV-Strahlungsquelle herum rotiert und spiralförmig weiter zur ersten Düsenstufe D1 strömt. Grundsätzlich könnte jedoch auch an der Einmündung zur ersten Behandlungskammer K1 eine Düsenstufe bereit gestellt werden.

Teilbereiche der Düsenstufen D1 bis D3 fungieren in diesem Beispiel gleichzeitig als Zentrierungseinrichtung 42 für die Quarzglasrohre T1 - T4. Die Düsenstufen D1 bis D3 sind zwischen zwei aufeinanderfolgenden Behandlungskammern abwechselnd am oberen und am unteren Deckel 36, 38 angeordnet, so dass in der Fig. 15 nur die zwei Düsenstufen D1 und D3 erkennbar sind. Die Düsenstufen D1 - D3 sind jeweils in Absätzen (oder auch Einbuchtungen oder dergleichen) an der Unterseite des oberen Deckels 36 und an der Oberseite des unteren Deckels 38 vorgesehen. Grundsätzlich können die Düsenstufen auch in nur einem Deckel angebracht sein.

Die Düsen 50 sind jeweils so ausgestaltet, dass ihre in eine jeweilige Behandlungskammer K2 - K4 mündende Düsenaustrittsöffnung eine vorbestimmte Ausstoßrichtung für das zu behandelnden Mediums definiert. Diese Ausstoßrichtung ist so gewählt, dass sie in Bezug zu der Ringform der Behandlungskammern K2 - K4 und zur Abstrahlungsrichtung R der UV-Strahlungsquelle im Wesentlichen tangential oder in einem dazu schrägen Winkel verläuft. Die Ausstoßrichtung der Düsen 50 kann zusätzlich auch eine in Längsrichtung L verlaufende Komponente aufweisen. Diese sollte jedoch vorzugsweise klein sein. Durch diese Maßnahmen wird das in die jeweilige Behandlungskammer K2 - K4 eingespritzte Medium in Rotation versetzt. Und das zu behandelnde Medium rotiert - wie in der Kammer K1 auch - innerhalb der Behandlungskammern K2 bis K4 kreis- bzw. spiralförmig um die UV-Strahlungsquelle herum.

Der Weg, den das zu behandelnde Medium innerhalb der Vorrichtung zurücklegt, wird auf diese Weise um ca. 480% pro Behandlungskammer K1 - K4 verlängert. Das Medium dreht sich in den Behandlungskammern K1 - K4 mit einer hohen Rotationsgeschwindigkeit um die UV-Strahlungsquelle herum, bis es schließlich die letzte Behandlungskammer K4 verlässt. Somit werden das Medium und darin ggf. enthaltene Verunreinigungen der Strahlung der UV-Strahlungsquelle länger ausgesetzt, was eine optimale Ausnutzung der Entkeimungs- und Oxidationsleistung gestattet.

Die Düsenaustrittsöffnungen und die Ausstoßrichtungen der Düsen 50 von zwei aufeinanderfolgenden Behandlungskammern K1-K2, K2-K3, K3-K4 sind vorzugsweise in entgegengesetzten Richtungen zueinander ausgerichtet, so dass sich die Rotationsrichtung des Mediums bei einem Übergang von einer Behandlungskammer zur nächsten jeweils umgekehrt. Auch dies trägt zur Verlängerung der Verweilzeit des Mediums in der Strahlung der UV-Strahlungsquelle bei und fördert somit eine verbesserte Ausnutzung der Entkeimungs- und Oxidationsleistung.

Zwischen zwei aufeinanderfolgenden Behandlungskammern K1-K2, K2-K3, K3-K4, bei denen der Düsenkanal 52 den Verbindungskanal bildet, ist zudem jeweils eine Drosseleinrichtung für das von einer Behandlungskammer K2 - K4 zur nächsten strömende, zu behandelnde Medium angeordnet. Diese Drosseleinrichtung verursacht durch einen definierten Rückstau des Mediums einen Druckanstieg in dem Medium und beaufschlagt die Düsen 50 mit einem vorbestimmten Druck, so dass das Medium mit einer hohen Beschleunigung und einer hohen Geschwindigkeit in die jeweilige Behandlungskammer K2, K3, K4 eingespritzt werden kann. Im vorliegenden Ausführungsbeispiel ist eine jeweilige Düse 50 an ihrer Medium-Eintrittsseite als Drosseleinrichtung ausgebildet, was durch eine geeignete Düsenform erreichbar ist.

Wie aus der Fig. 15 des Weiteren hervorgeht, ist der den jeweiligen Verbindungskanal bildende, sich in Ausstoßrichtung verjüngende Düsenkanal 52 einer Düse 50 bogenförmig ausgebildet bzw. besitzt bogenförmige oder parabolisch gekrümmte Düsenkanal-Wandungen.

Eine jeweilige Düse 50 ist darüber hinaus an ihrer Düsenaustrittsöffnung mit einer kleinen Unstetigkeitsstelle ausgestattet (in den Figuren nicht erkennbar), welche innerhalb des aus der Düse 50 ausströmenden Mediums hochfrequente kleine Wirbel erzeugt. Bei einer Unstetigkeitsstelle kann es sich z.B. um eine Abrisskante bzw. -schwelle am Düsenausgang, einen asymmetrischen Düsenaustritt oder auch um eine vibrierende Düsenzunge handeln. Bei Verwendung einer vibrierenden Düsenzunge kann diese passiv (z.B. durch einen infolge des strömenden Mediums hervorgerufenen Resonanzeffekt) oder aktiv (z.B. durch einen Antrieb) in Schwingungen versetzt werden. Die hochfrequenten kleinen Wirbel mindern ähnlich dem sog. Haifischhauteffekt die Reibung des strömenden Mediums an den Wänden der Behandlungskammern K2-K4, wodurch ein übermäßiger Druckverlust innerhalb der Behandlungskammern K2-K4 vermieden werden kann. Dies ist wichtig, weil der definierte Rückstau des Medium zur Druckbeaufschlagung der Düsen 50 ja durch die Form der als Verbindungskanäle fungierenden Düsenkanäle 52 erzeugt werden soll und nicht durch einen unzulässig großen reibungsbedingten Druckverlust gemindert werden darf.

Wenn das zu behandelnde Medium Wasser ist, so bewirken die Düsen der erfindungsgemäßen Vorrichtung gemäß der siebten Ausführungsform einen weiteren positiven Effekt. Bekanntlich neigen Wassermoleküle bei statischen Druck, wie z.B. in Wasserleitungen, zur Cluster-Bildung, wodurch sich die Lösungseigenschaften des Wassers reduzieren. Durch die mechanischen Kräfte des Düseneffektes werden die Cluster der Wassermoleküle wieder zerkleinert, wodurch die natürlichen Lösungseigenschaften des Wasser wiederhergestellt werden.

Die Erfindung ist nicht auf die obigen Ausführungsbeispiele beschränkt, die lediglich der allgemeinen Erläuterung des Kerngedankens der Erfindung dienen. Im Rahmen des Schutzumfangs kann die erfindungsgemäße Vorrichtung vielmehr auch andere als die oben konkret beschriebenen Ausgestaltungsformen annehmen. So können die Behandlungskammern beispielsweise auch transparente, UV-durchlässige Trennschichten aufweisen, die pro einzelner Trennschicht unterschiedliche Durchlässigkeiten bzw. Transmissionseigenschaften für unterschiedliche UV-Strahlungsspektren bzw. Teilspektren aufweisen. Obwohl in den obigen Beispielen natürliches oder synthetisches Quarzglas für die Trennschichten verwendet wurde, können grundsätzlich auch andere UV-durchlässige Materialien Anwendung finden, wenn diese die für jeweils benötigten Wellenlängenbereich der UV-Strahlung die oben beschriebenen Eigenschaften aufweisen. Je nach Anwendungsfall kann die Anzahl der Behandlungskammer variieren, die beträgt jedoch mindestens zwei.

Die transparenten, UV-durchlässigen und als optische Linse bzw. Lichtleiter ausgestalteten Verwirbelungselemente 28 können zudem so geformt werden, dass sie ihre gewünschte optische Wirkung bei Strahlungseinfall von beiden Seiten einer jeweiligen Trennebene her entfalten. Auf diese Weise kann die Vorrichtung nicht nur mit einer zentralen UV-Strahlungsquelle, sondern z.B. auch mit um die jeweiligen Behandlungskammern herum platzierten zusätzlichen UV-Strahlungsquellen ausgestattet werden und die oben beschriebenen gewünschten Wirkungen erzielen.

Zusätzlich zu oder alternativ zu den obigen Ausführungsbeispielen nach **Fig. 10 bis 11** kann auch eine transparente, UV-durchlässige Trennschicht Bereiche aufweisen, die als optische Linse bzw. Lichtleiter ausgebildet sind. Diese Bereiche müssen nicht zwangsläufig auch die Wirbelelemente 28 bilden oder als solche fungieren. Diese Variante ist selbstverständlich auch in Kombination mit den oben beschriebenen transparenten und UV-durchlässigen, als optische Linsen und/oder Lichtleiter verwendeten Verwirbelungselementen realisierbar.

Bezugszeichen in den Ansprüchen, der Beschreibung und den Zeichnungen dienen lediglich dem besseren Verständnis der Erfindung und sollen den Schutzumfang nicht einschränken.

### Bezugszeichenliste

Es bezeichnen:
- 2: Reaktor
- 4: Einspeisungskanal
- 6: Auslasskanal
- 8: Kontrolleinrichtung
- 10: Sensor
- 12: Sensor
- 14: Vorfiltereinrichtung
- 16: Pumpe
- 18: Leitung
- 20: Magnetventil
- 22: Nachgeschaltete Filtereinrichtung
- 24: UV-Strahlungsquelle
- 26: Austrittsöffnung / Eintrittsöffnung
- 28: Verwirbelungselemente
- 30: UV-Reflektionseinrichtung
- 32: UV-Reflektoreinrichtung
- 34: Trennende UV-Reflektionseinrichtung
- 36: Oberer Deckel
- 38: Unterer Deckel
- 40: Durchgangsöffnungen
- 42: Zentrierungseinrichtung
- 44: Einlass
- 46: Auslass
- 48: Halbkreisförmiger Kanal
- 50: Düse (n)
- 52: Düsenkanal / Verbindungskanal

- D1 - D4: Düseneinrichtung / Düsenstufen

- K1 - K4: Behandlungskammern
- KL: Letzte bzw. äußerste Wandung
- L: Längsachse
- R: Abstrahlrichtung von 24
- T1, T2: Transparente, UV-durchlässige Trennschicht aus synthetischem Quarzglas
- T3, T4: Transparente, UV-durchlässige Trennschicht aus natürlichem Quarzglas
- T5: Verspiegeltes Quarzglasrohr

## Patentansprüche

1. Vorrichtung zur Behandlung eines flüssigen oder gasförmigen Mediums, insbesondere Wasser oder Luft, mittels UV-Strahlen, umfassend:
- eine UV-Strahlungsquelle (24) mit einer axialen Längserstreckung und einer dazu Im Wesentlichen senkrechten, insbesondere radialen Abstrahlungsrichtung (R); und
- mehrere Schichten von in Abstrahlungsrichtung (R) übereinander angeordneten, aufeinanderfolgenden, durchstrahlbaren Behandlungskammern (K1 - K4), die
von der UV-Strahlungsquelle (24) und voneinander jeweils durch eine transparente, UV-durchlässige Trennschicht (T1 - T4) getrennt sind,
und die,
beginnend bei einer der UV-Strahlungsquelle (24) in Abstrahlungsrichtung nächstliegenden, ersten Behandlungskammer
jeweils einen entlang der Längserstreckung der UV-Strahlungsquelle (24) verlaufenden Durchströmungskanal für das Medium bilden, der in die jewells nachfolgende, von der UV-Strahlungsquelle (24) in Abstrahlungsrichtung (R) weiter entfernte Behandlungskammer (K2, K3, K4) mündet (26), **dadurch gekennzeichnet, dass** transparente und UV-durchlässige Verwirbelungselemente (28) Bestandteil einer transparenten, UV-durchlässigen Trennschicht (T1; T2; T3, T4) sind und dass die transparenten und UV-durchlässigen Verwirbelungselemente (28) jeweils als optische Linse ausgebildet sind.

2. Vorrichtung nach Anspruch 1,
**dadurch gekennzeichnet, dass**
die UV-Strahlungsquelle (24) ein UV-Strahlungsspektrum von mindestens 180 nm bis zumindest 254 nm Wellenlänge besitzt.

3. Vorrichtung nach Anspruch 1 oder 2,
**dadurch gekennzeichnet dass**
die mehreren Schichten von Behandlungskammern (K1 - K4) in radialer Richtung (R) um die UV-Strahlungsquelle (24) herum angeordnet sind.

4. Vorrichtung nach einem oder mehreren der vorhergenannten Ansprüche
**dadurch gekennzeichnet, dass**
die mehreren Schichten von Bahandlungskammern (K1 - K4) konzentrisch um die UV-Strahlungsquelle (24) herum angeordnet sind.

5. Vorrichtung nach einem oder mehreren der vorhergenannten Ansprüche
**dadurch gekennzeichnet, dass**
sich mindestens eine der Behandlungskammern (K1 - K4) der mehreren Schichten von Behandlungskammern (K2 - K4) spiralförmig um die UV-Strahlungsquelle (24) herum ersteckt.

6. Vorrichtung nach einem oder mehreren der vorher genannten Ansprüche,
**dadurch gekennzeichnet, dass**
ausgehend von der UV-Strahlungsquelle (24) in Abstrahlungsrichtung (R) mindestens die erste (T1) der transparenten UV-durchhlässigen Trennschichten (T1 - T4), die zwischen der UV-Strahlungsquelle (24) und der ersten Behandlungskammer (K1) angeordnet ist und der UV-Strahlungsquelle (24) am nächsten liegt, aus einem ersten Trennschicht-Material hergestellt ist, welches eine UV-Durchlässigkeit besitzt, die im Wesentlichen das gesamte UV-Strahlungsspektrum der UV-Strahlungsquelle (24) einschließlich eines ersten Tellspektrums mit Wellenlängen kleiner 200nm, Insbesondere einen Wellenlängenbereich kleiner, 200nm und größer gleich 180nm,
durchlässt.

7. Vorrichtung nach Anspruch 6
**dadurch gekennzeichnet, dass**
wenigstens eine weitere (T2) der Trennschichten (T1 - T4), welche der ersten Trennschicht (T1) in Abstrahlungsrichtung (R) nachfolgt, aus dem ersten Trennschicht-Material hergestellt ist.

8. Vorrichtung nach Anspruch 6 oder 7,
**dadurch gekennzeichnet, dass**
die in Abstrahlungsrichtung (R) gemessene Gesamtdicke einer Teilschicht,
die aus einer oder mehreren Trennschichten (T1, T2) aus dem erstem Trennschicht-Material und einer oder mehreren Behandlungskammern (K1, K2) gebildet wird, in welche die UV-Strahlung durch das erste Trennschicht-Material eintritt,
im Wesentlichen einer maximalen effektiven Eindringtiefe des ersten Teilspektrums der UV-Strahlung entspricht,
die sich in Abhängigkeit der Absorptionsfähigkeit des jeweils zu behandelnden Mediums und der UV-Durchlässigkeit des ersten Trennschicht-Materials für dieses erste Tellspektrum ergibt.

9. Vorrichtung nach einem der Ansprüche 6 bis 8,
**dadurch gekennzeichnet, dass**
mindestens eine weitere (T3, T4) der Trennschichten (T1 - T4), welche in Abstrahlungsrichtung einer Trennschicht (T1, T2) nachfolgt, die aus dem ersten Trennschicht-Material hergestellt ist aus einem zweiten Trennschicht-Material gefertigt ist, welches eine UV-Durchlässigkeit besitzt, die von dem UV-Strahlungsspektrum der UV-Strahlungsquelle (24) nur UV-Strahlung in einem zweiten Teilspektrum mit Wellenlängen größer gleich 200nm durchlässt. '

10. Vorrichtung nach einem oder mehreren der vorhergenannten Ansprüche
**dadurch gekennzeichnet, dass**
diese eine Verweilzeit-Verlängerungseinrichtung zum vertängern der Verweilzeit des zu behandelnden Mediums im Strahlungsbereich der UV-Strahlungsquelle (24) besitzt.

11. Vorrichtung nach Anspruch 10,
**dadurch gekennzeichnet dass**
die Verweilzeit-Verlängerungseinrichtung einen Einspeisungskanal (4) für das zu behandelnde Medium besitzt, der bezogen auf die Abstrahlungsrichtung (R) im Wesentlichen tangential in die erste Behandlungskammer (K1) mündet und dem einzuspeisenden Medium einen Drall verleiht.

12. Vorrichtung nach Anspruch 10 oder 11,
**dadurch gekennzeichnet, dass**
die Verweilzeit-Verlängerungseinrichtung einen Auslasskanal (4) für das behandelte Medium besitzt, der bezogen auf die Abstrahlungsrichtung (R) im Wesentlichen tangential und/oder in Drallrichtung aus der letzten, der UV-Strahlungsquelle (24) in Abstrahlungsrichtung (R) am weitesten entfernten Behandlungskammer (K4) heraustritt.

13. Vorrichtung nach einem oder mehreren der vorher genannten Ansprüche,
**dadurch gekennzeichnet, dass**
die Verweilzeit-Verlängerungseinrichtung Verwirbelungselemente (28) aufweist, die in Vorrichtungskomponenten angeordnet sind, die ausgewählt sind aus einer Gruppe von Vorrichtungskomponenten, umfassend: die Behandlungskammern (K1 - K4), den Einspeisungskanal (4), den Auslasskanal (6), Eintritts- oder Austrittsöffnungen (26) zwischen aufeinanderfolgenden Behandlungskammern (K1 - K4).

14. Vorrichtung nach einem oder mehreren der vorher genannten Ansprüche,
**dadurch gekennzeichnet, dass**
die aufeinanderfolgenden Behandlungskammern (K1 - K4) einen Teil der Verweilzeit-Verlängerungseinrichtung bilden.

15. Vorrichtung nach einem oder mehreren der vorher genannte Ansprüche,
**dadurch gekennzeichnet, dass**
die letzte der Behandlungskammern (K4) an ihrer von der UV-Strahlungsquelle in Abstrahlungsrichtung am weitesten entfernten Seite eine UV-Reflektionseinrichtung (30; 32) besitzt.

16. Vorrichtung nach einem oder mehreren der vorher genannten Ansprüche,
**dadurch gekennzeichnet, dass**
die Verwirbelungselemente (28) der Verweilzeit-Verlängerungseinrichtung transparent und UV-durchlässig ausgebildet sind.

17. Vorrichtung nach einem oder mehreren der vorher genannten Ansprüche,
**dadurch gekennzeichnet dass**
die transparente, UV-durchlässige Trennschicht (T1; T2; T3, T4) Bereiche aufweist, die als optische Linse ausgebildet sind.

18. Vorrichtung nach einem oder mehreren der vorher genannten Ansprüche,
**Dadurch gekennzeichnet, dass**,
dreidimensional verformte Bereiche einer transparenten, UV-durchlässigen Trennschicht, (T1; T2; T3, T4) die transparenten, UV-durchlässigen und als optische Linse oder Lichtleiter ausgebildeten Verwirbelungselemente (28) bilden.

19. Vorrichtung nach einem oder mehreren der vorher genannten Ansprüche,
**dadurch gekennzeichnet, dass**
eine optische Ausgangsebene (bzw. Eingangsebene) eines als optische Linse ausgebildeten Verwirbelungselementes (28) einer ersten Trennschicht (z.B. T1) in einem Abstand von einer optischen Eingangsebene (bzw. Ausgangsebene) eines als optische Linse ausgebildeten Verwirbelungselementes (28) einer In Abstrahlungsrichtung (R) nachfolgenden, zweiten Trennschicht (z.B. T2) angeordnet ist, der kleiner gleich einem Abstand ist, der ausgewählt ist aus einer Gruppen von Abständen, umfassend: kleiner gleich 10 mm, kleiner gleich 9 mm, kleiner gleich 8 mm, kleiner gleich 7 mm, kleiner gleich 6 mm, kleiner gleich 5 mm, kleiner gleich 4 mm, kleiner gleich 3 mm, kleiner gleich 2 mm, kleiner gleich 1 mm, kleiner gleich 0,5 mm, kleiner gleich 0,25 mm.

20. Vorrichtung nach Anspruch 19,
**dadurch gekennzeichnet, dass**
die optische Eingangsebene (bzw. Ausgangsebene) eines als optische Linse ausgebildeten Verwirbelungselementes (28) einer in Abstrahlungsrichtung (R) von der UV-Strahlungsquelle distal angeordneten Trennschicht (z.B. T2) in einem Abstand von der optischen Ausgangsebene (bzw. Eingangsebene) einer in Abstrahlungsrichtung (R) von der UV-Strahlungsquelle proximal angeordneten Trennschicht (z.B. T1) angeordnet ist, der kleiner gleich einem Abstand ist, der ausgewählt ist aus einer Gruppen von Abständen, umfassend: kleiner gleich 10 mm, kleiner gleich 9 mm, kleiner gleich 8 mm, kleiner gleich 7 mm, kleiner gleich 6 mm, kleiner gleich 5 mm, kleiner gleich 4 mm, kleiner gleich 3 mm, kleiner gleiche 2 mm, kleiner gleich 1 mm, kleiner gleich 0,5 mm, kleiner gleich 0,25 mm.

21. Vorrichtung nach einen oder mehreren der vorher genannten Anspruche,
**dadurch gekennzeichnet, dass**
diese einen oberen (36) und einen unteren Deckel (38) aufweist, zwischen denen die UV-Strahlungsquelle (24) und die die Behandlungskammern (K1 - K4) voneinander trennenden UV-durchlässigen Trennschichten (T1 - T4) angeordnet sind, und mindestens einer der Deckel (36, 38) einen mit mindestens einer ersten der Behandlungskammern (K1 - K4) kommunizierenden Einlass (44) und/oder Auslass (46) für das zu behandelnde Medium besitzt.

22. Vorrichtung nach Anspruch 21,
**dadurch gekennzeichnet, dass**
die Deckel (36, 38) jeweils eine Zentrierungseinrichtung (40; 42; D1-D3) für die dazwischen angeordneten UV-durchlässigen Trennschichten (T1 - T4) aufweisen.

23. Vorrichtung nach Anspruch 21 oder 22,
**dadurch gekennzeichnet, dass**
mindestens einer der Deckel (36, 38) wenigsten einen Verbindungskanal (52) aufweist, welche eine jeweilige Behandlungskammer (K1 - K3) mit der jeweils nachfolgende Behandlungskammer (K2 - K4) verbindet.

24. Vorrichtung nach einem der Ansprüche 21 bis 23,
dadurch gekenntzeichnet, dass
zumindest ein Teil (50, 52; D1-D3) der Verweilzeit-Verlängerungseinrichtung in mindestens einem der Deckel (36, 38) angeordnet ist.

25. Vorrichtung nach einem der Ansprüche 21 bis 24,
**dadurch gekennzeichnet, dass**
der in mindestens einem der Deckel (36, 38) angeordnete Teil der verweilzeit-Verlängerungseinrichtung eine Verwirbelungseinrichtung (50, 52; D1-D3) für das zu behandelnde Medium besitzt.

26. Vorrichtung nach einem der Ansprüche 23 bis 25,
**dadurch gekennzeichnet, dass**
die Verwirbelungseinrichtung in den mindestens einen Verbindungskanal (52) integriert ist.

27. Vorrichtung nach einem oder mehreren der vorher genannten Ansprüche,
**dadurch gekennzeichnet, dass**
die Verwirbelungseinrichtung eine Düseneinrichtung (D1-D3) mit mindesten einer Düse (50) ausweist.

28. Vorrichtung nach Anspruch 27,
**dadurch gekennzeichnet, dass**
die Düse (50) eine in eine Behandlungskammer (K1 - K4) mündende Düsenaustrittsöffnung aufweist, welche in Bezug zu der Abstrahlungsrichtung (R) der UV-Strahlungsraquelle eine in einem achrägen oder tangentialen winkel verlaufende Ausstoßrichtung des zu behandelnden Mediums definiert und das in die jeweiligen Behandlungskanmler (K2 - K4) einströmende Medium in Rotation versetzt.

29. Vorrichtung nach Anspruch 28,
**dadurch gekennzeichnet, dass**
die Düsenaustrittsöffnungen und die Ausstoßrichtungen der Düsen . (50) von zwei aufeinanderfolgenden Behandlungskammern (K1-K2; K2-K3; K3-K4) in entgegengesetzten Richtungen zueinander ausgerichtet sind, so dass sich die Rotationsrichtung des Mediums bei einem Übergang von einer Behandlungakammer zur nächsten umgekehrt..

30. Vorrichtung nach Anspruch 28 oder 29,
**dadurch gekennzeichnet, dass**
die mindestens ein Düse (50) an ihrer Düsenaustrittsöffnung eine Unstetigkeitsstelle aufweist, welche das aus der Düse (50) ausströmende Medium in hochfrequente Wirbel versetzt.

31. Vorrichtung nach einem oder mehreren der vorher genannten Ansprüche,
**dadurch gekennzeichnet, dass**
zwischen jeweils zwei aufeinanderfolgenden Behandlungskammern (K1-K2, K2-K3; K3-K4) eine Vielzahl von Düsen (50; D1-D3) vorgesehen ist, welche bezogen auf eine Längsachse (L) der Behandlungskammern (K1 - K4) oder der UV-Strahlungsquelle kranzartig um die Längsachse (L) herum angeordnet sind.

32. Vorrichtung nach einem der Ansprüche 23 bis 31,
**dadurch gekennzeichnet, dass**
zwischen zwei aufeinanderfolgenden und durch den Verbindungskanal (52) miteinander verbundenen Behandlungskammern (K1-K2; K2-K3; K3-K4) mindestens eine Drosseleinrichtung (50) für das von einer Behandlungskammer zur nächsten strömende zu behandelnde Medium angeordnet ist, .welche (50) einen Druckanstieg in dem Medium verursacht und die Verwirbelungseinrichtung (50; D1-D3) mit einem vorbestimmten Druck des zu behandelnden Mediums beaufschlagt.

33. Verwendung der Vorrichtung nach einem oder mehreren der Ansprüche 1 bis 35 zur kombinierten UV-Entkeimung und UV-Oxidation, insbesondere zur Wasseraufbereitung.

## Claims

1. Device for the treatment of a liquid or gaseous medium, in particular water or air, by means of UV radiation, comprising:
- a UV radiation source (24) with an axial longitudinal extension and a direction of radiation (R) which is substantially perpendicular, in particular radial, thereto; and
- a plurality of layers of treatment chambers (K1 - K4) serially arranged on top of each other in the direction of radiation (R) through which the radiation can pass, which
are each separated from the UV radiation source (24) and from each other by means of a transparent, UV-permeable separating layer (T1 - T4),
and which,
starting from a first treatment chamber (K1), which is nearest to the UV radiation source (24) in the direction of radiation (R),
each form a flow channel for the medium running along the longitudinal extension of the UV radiation source (24), with said flow channel opening into the respective adjacent treatment chamber (K2, K3, K4) which is farther from the UV radiation source (24) in the direction of radiation (R) (26),
**characterized in that**
transparent and UV-permeable turbulence elements (28) form part of a transparent, UV-permeable separating layer (T1; T2; T3, T4)
and that
the transparent and UV-permeable turbulence elements (28) are each configured in the form of an optical lens.

2. Device according to claim 1,
**characterized in that**
the UV radiation source (24) has a UV radiation spectrum with a wavelength ranging from at least 180 nm to at least 254 nm.

3. Device according to claim 1 or 2,
**characterized in that**
the plurality of layers of treatment chambers (K1 - K4) is arranged around the UV radiation source (24) in the radial direction (R).

4. Device according to one or more of the preceding claims,
**characterized in that**
the plurality of layers of treatment chambers (K1 - K4) is arranged concentrically around the UV radiation source (24).

5. Device according to one or more of the preceding claims,
**characterized in that**
at least one of the treatment chambers (K1 to K4) of the plurality of layers of treatment chambers (K1 - K4) spirals around the UV radiation source (24).

6. Device according to one or more of the preceding claims,
**characterized in that**,
starting from the UV radiation source (24) in the direction of radiation (R), at least the first (T1) of the transparent, UV-permeable separating layers (T1 - T4), which is arranged between the UV radiation source (24) and the first treatment chamber (K1) and which is nearest to the UV radiation source (24), is made of a first separating layer material having a permeability to UV radiation which substantially allows the entire UV radiation spectrum of the UV radiation source (24) to pass, including a first partial spectrum with wavelengths shorter than 200 nm and longer than or equal to 180 nm.

7. Device according to claim 6,
**characterized in that**
at least one further separating layer (T2) of the separating layers (T1 - T4) which is arranged in the direction of radiation (R) adjacent to the first separating layer (T1) is made of the first separating layer material.

8. Device according to claim 6 or 7,
**characterized in that**
the total thickness of one partial layer measured in the direction of radiation (R)
which is formed from one or a plurality of separating layers (T1, T2) made of the first separating layer material and one or a plurality of treatment chambers (K1, K2), into which the UV radiation penetrates via the first separating layer material,
substantially corresponds to a maximum effective penetration depth of the first partial spectrum of the UV radiation
resulting for said first partial spectrum as a function of the absorptive capacity of the respective medium to be treated and the permeability to UV radiation of the first separating layer material.

9. Device according one of claims 6 to 8,
**characterized in that**
at least one further separating layer (T3, T4) of the separating layers (T1 - T4) which is arranged in the direction of radiation (R) adjacent to one separating layer (T1, T2) made of the first separating layer material is made of a second separating layer material having a permeability to UV radiation which only allows UV radiation out of the UV radiation spectrum of the UV radiation source (24) to pass within a second partial spectrum with wavelengths longer or equal to 200 nm.

10. Device according to one or more of the preceding claims,
**characterized in that**
said device has a sojourn time extension device for extending the sojourn time of the medium to be treated within the range of radiation of the UV radiation source (24).

11. Device according to claim 10,
**characterized in that**
the sojourn time extension device has a feed channel (4) for the medium to be treated which, relative to the direction of radiation (R), substantially tangentially opens into the first treatment chamber (K1) and induces a swirling motion of the medium to be fed in.

12. Device according to claim 10 or 11,
**characterized in that**
the sojourn time extension device has an outlet channel (6) for the medium treated which, relative to the direction of radiation (R), substantially tangentially and/or in the direction of swirl exits the last treatment chamber (K4), which is farthest from the UV radiation source (24) in the direction of radiation (R).

13. Device according to one or more of the preceding claims,
**characterized in that**
the sojourn time extension device has turbulence elements (28) which are arranged in device components selected from a group of device components, comprising: the treatment chambers (K1 - K4); the feed channel (4), the outlet channel (6), inlet or outlet openings (26) between serially arranged treatment chambers (K1 - K4).

14. Device according to one or more of the preceding claims,
**characterized in that**
the serially arranged treatment chambers (K1 - K4) form part of the sojourn time extension device.

15. Device according to one or more of the preceding claims,
**characterized in that**
the last treatment chamber (K4) has a UV reflection device (30; 32) on the side which is farthest from the UV radiation source (24) in the direction of radiation (R).

16. Device according to one or more of the preceding claims,
**characterized in that**
the turbulence elements (28) of the sojourn time extension device are configured to be transparent and UV-permeable.

17. Device according to one or more of the preceding claims,
**characterized in that**
the transparent, UV-permeable separating layer (T1; T2; T3, T4) has regions which are configured in the form of an optical lens.

18. Device according to one or more of the preceding claims,
**characterized in that**
three-dimensionally shaped regions of a transparent, UV-permeable separating layer (T1; T2; T3, T4) form the transparent, UV-permeable turbulence elements (28) configured in the form of an optical lenses or a fibre-optic light guides.

19. Device according to one or more of the preceding claims,
**characterized in that**
an optical output level (or input level) of a turbulence element (28) of a first separating layer (e.g. T1) configured in the form of an optical lens is arranged at a distance from an optical input level (or output level) of a turbulence element (28) of a second separating layer (e.g. T2) configured in the form of an optical lens, with said second separating layer adjoining in the direction of radiation (R), wherein the distance is less than or equal to a distance which is selected from a group of distances, comprising: less than or equal to 10 mm, less than or equal to 9 mm, less than or equal to 8 mm, less than or equal to 7 mm, less than or equal to 6 mm, less than or equal to 5 mm, less than or equal to 4 mm, less than or equal to 3 mm, less than or equal to 2 mm, less than or equal to 1 mm, less than or equal to 0,5 mm, less than or equal to 0,25 mm.

20. Device according to claim 19,
**characterized in that**
the optical input level (or output level) of a turbulence element (28) of a separating layer (e.g. T2) configured in the form of an optical lens, with said separating layer being disposed distally from the UV radiation source (24) in the direction of radiation (R), is arranged at a distance from the optical output level (or input level) of a separating layer (e.g. T1) being disposed proximally from the UV radiation source (24) in the direction of radiation (R), wherein the distance is less than or equal to a distance which is selected from a group of distances, comprising: less than or equal to 10 mm, less than or equal to 9 mm, less than or equal to 8 mm, less than or equal to 7 mm, less than or equal to 6 mm, less than or equal to 5 mm, less than or equal to 4 mm, less than or equal to 3 mm, less than or equal to 2 mm, less than or equal to 1 mm, less than or equal to 0,5 mm, less than or equal to 0,25 mm.

21. Device according to one or more of the preceding claims,
**characterized in that**
said device has a top (36) and a bottom cover (38) between which the UV radiation source (24) and the UV-permeable separating layers (T1 - T4) separating the treatment chambers (K1 - K4) from each other are arranged and that at least one of the covers (36, 38) has an inlet (44) and/or outlet (46) for the medium to be treated, communicating with at least a first one of the treatment chambers (K1 - K4).

22. Device according to claim 21,
**characterized in that**
the covers (36, 38) each have a centring device (40, 42; D1 - D3) for the UV-permeable separating layers (T1 - T4) arranged in between.

23. Device according to claim 21 or 22,
**characterized in that**
at least one of the covers (36, 38) has at least one connection duct (52) connecting a respective treatment chamber (K1 - K3) to the respectively adjacent treatment chamber (K2 - K4).

24. Device according to one of claims 21 to 23,
**characterized in that**
at least one part (50, 52; D1 - D3) of the sojourn time extension device is arranged in at least one of the covers (36, 38).

25. Device according to one of claims 21 to 24,
**characterized in that**
the part of the sojourn time extension device arranged in at least one of the covers (36, 38) has a turbulence device (50, 52; D1 - D3) for the medium to be treated.

26. Device according to one of claims 23 to 25,
**characterized in that**
the turbulence device is integrated in the at least one connection duct (52).

27. Device according to one or more of the preceding claims,
**characterized in that**
the turbulence device has a nozzle device (D1 - D3) with at least one nozzle (50).

28. Device according to claim 27,
**characterized in that**
the nozzle (50) has a nozzle outlet which opens into a treatment chamber (K1 - K 4) and which, relative to the direction of radiation (R) of the UV radiation source, defines a direction of discharge of the medium to be treated running at an oblique or tangential angle and which causes the medium flowing into the respective treatment chamber (K2 - K4) to rotate.

29. Device according to 28,
**characterized in that**
the nozzle outlets and the directions of discharge of the nozzles (50) of two serially arranged treatment chambers (K1 - K2; K2 - K3; K3 - K4) are aligned with each other in opposite directions such that the direction of rotation of the medium is reversed when the latter passes from one treatment chamber to the next.

30. Device according to claim 28 or 29,
**characterized in that**,
at the nozzle outlet, the at least one nozzle (50) has a point of discontinuity causing high-frequency turbulences in the medium flowing out of the nozzle (50).

31. Device according to one or more of the preceding claims,
**characterized in that**,
between two serially arranged treatment chambers (K1 - K2; K2 - K3; K3 - K4), a plurality of nozzles (50; D1 - D3) is in each case provided which, relative to a longitudinal axis (L) of the treatment chambers (K1 - K4) or the UV radiation source, are annularly arranged around the longitudinal axis (L).

32. Device according to one of claims 23 to 31,
**characterized in that**,
between two serially arranged treatment chambers (K1 - K2; K2 - K3; K3 - K4) being connected to each other via the connection duct (52), at least one throttling device (50) for the medium to be treated flowing from one treatment chamber to the next is arranged, with said throttling device (50) causing an increase in pressure in the medium and applying a predetermined pressure of the medium to be treated to the turbulence device (50; D1 - D3).

33. Use of the device according one or more of claims 1 to 35 for combined UV sterilisation and UV oxidation, in particular for the treatment of water.

## Revendications

1. Dispositif pour le traitement d'une substance liquide ou gazeuse, en particulier de l'eau ou de l'air, au moyen de rayons UV, comprenant:
- une source de rayonnement UV (24) avec une extension longitudinale axiale et une direction de rayonnement (R) sensiblement perpendiculaire à celle-ci, en particulier radiale ; et
- plusieurs couches de chambres de traitement (K1 - K4) irradiables, successives et agencées les unes au-dessus des autres en direction de rayonnement, qui
sont séparées de la source de rayonnement UV (24) et les unes des autres par une couche de séparation (T1-T4) respective transparente et perméable aux UV,
et qui
en partant d'une première chambre de traitement (K1) la plus proche de la source de rayonnement UV (24) en direction de rayonnement
forment chacune un canal d'écoulement pour la substance, qui s'étend le long de l'extension longitudinale de la source de rayonnement UV (24), et qui débouche (26) dans la chambre de traitement (K2, K3, K4) respective suivante plus éloignée de la source de rayonnement UV (24) en direction de rayonnement,
**caractérisé en ce que** des éléments tourbillonnaires (28) transparents et perméables aux UV font partie d'une couche de séparation (T1 ; T2 ; T3 ; T4) transparente et perméable aux UV, et **en ce que** les éléments tourbillonnaires (28) transparents et perméables aux UV sont réalisés chacun sous forme de lentille optique.

2. Dispositif selon la revendication 1, **caractérisé en ce que** la source de rayonnement UV (24) possède un spectre de rayonnement UV d'au moins 180 nm jusqu'à au moins 254 nm de longueur d'onde.

3. Dispositif selon la revendication 1 ou 2, **caractérisé en ce que** les plusieurs couches de chambres de traitement (K1 - K4) sont agencées en direction radiale (R) autour de la source de rayonnement UV (24).

4. Dispositif selon l'une ou plusieurs des revendications précédentes, **caractérisé en ce que** les plusieurs couches de chambres de traitement (K1 - K4) sont agencées de manière concentrique autour de la source de rayonnement UV (24).

5. Dispositif selon l'une ou plusieurs des revendications précédentes, **caractérisé en ce qu'**au moins une des chambres de traitement (K1 - K4) des plusieurs couches de chambres de traitement (K1 - K4) sont agencées en forme de spirale autour de la source de rayonnement UV (24).

6. Dispositif selon l'une des revendications précédentes, **caractérisé en ce qu'**en partant de la source de rayonnement UV (24) en direction de rayonnement, au moins la première (T1) des couches de séparation (T1 -T4) transparentes perméables aux UV, qui est agencée entre la source de rayonnement (24) et la première chambre de traitement (K1) et qui est la plus proche de la source de rayonnement (24), est fabriquée dans un matériau de couche de séparation qui possède une perméabilité aux UV qui laisse passer sensiblement tout le spectre de rayonnement UV de la source de rayonnement (24), y compris un premier spectre partiel avec des longueurs d'ondes inférieures à 200 nm, en particulier une plage de longueur d'onde inférieure à 200 nm et supérieure ou égale à 180 nm.

7. Dispositif selon la revendication 6, **caractérisé en ce qu'**au moins une autre (T2) des couches de séparation (T1 - T4), qui succède à la première couche de séparation (T1) en direction de rayonnement (R), est fabriquée dans le premier matériau de couche de séparation.

8. Dispositif selon la revendication 6 ou 7, **caractérisé en ce que** l'épaisseur totale d'une couche partielle, mesurée en direction de rayonnement (R),
qui est formée à partir d'une ou de plusieurs couches de séparation (T1, T2) dans le premier matériau de couche de séparation et d'une ou de plusieurs chambres de traitement (K1, K2) dans lesquelles le rayonnement UV entre à travers le premier matériau de couche de séparation,
correspond sensiblement à une profondeur de pénétration maximale effective du premier spectre partiel du rayonnement UV,
qui est obtenue pour ce premier spectre partiel en fonction du pouvoir absorbant de la substance respective à traiter et de la perméabilité aux UV du premier matériau de couche de séparation.

9. Dispositif selon l'une des revendications 6 à 8, **caractérisé en ce qu'**au moins une autre (T3, T4) des couches de séparation (T1 - T4) qui succède, en direction de rayonnement, à une couche de séparation (T1, T2) fabriquée dans le premier matériau de couche de séparation, est réalisée en un deuxième matériau de couche de séparation qui possède une perméabilité aux UV qui, du spectre de rayonnement UV de la source de rayonnement UV (24), ne laisse passer que du rayonnement UV dans un deuxième spectre partiel avec des longueurs d'ondes supérieures ou égales à 200 nm.

10. Dispositif selon l'une des revendications précédentes, **caractérisé en ce que** celui-ci possède un dispositif de prolongement du temps d'exposition pour prolonger le temps d'exposition de la substance à traiter dans la plage de rayonnement de la source de rayonnement UV (24).

11. Dispositif selon la revendication 10, **caractérisé en ce que** le dispositif de prolongement du temps d'exposition possède un canal d'alimentation (4) pour la substance à traiter qui, par rapport à la direction de rayonnement (R), débouche sensiblement tangentiellement dans la première chambre de traitement (K1) et confère un mouvement giratoire à la substance à injecter.

12. Dispositif selon la revendication 10 ou 11, **caractérisé en ce que** le dispositif de prolongement du temps d'exposition possède un canal d'évacuation (6) pour la substance traitée qui, par rapport à la direction de rayonnement (R) sort sensiblement tangentiellement et/ou en direction giratoire de la dernière chambre de traitement (K4) qui est la plus éloignée de la source de rayonnement UV (24) en direction de rayonnement (R).

13. Dispositif selon l'une ou plusieurs des revendications précédentes, **caractérisé en ce que** le dispositif de prolongement du temps d'exposition présente des éléments de tourbillonnement (28) qui sont agencés dans des composants de dispositif qui sont choisis parmi un groupe de composants de dispositif comprenant : les chambres de traitement (K1 - K4), le canal d'alimentation (4), le canal d'évacuation (6), des orifices d'entrée et de sortie (26) entre des chambres de traitement (K1 - K4) successives.

14. Dispositif selon l'une ou plusieurs des revendications précédentes, **caractérisé en ce que** les chambres de traitement (K1 - K4) successives forment une partie du dispositif de prolongement du temps d'exposition.

15. Dispositif selon l'une ou plusieurs des revendications précédentes, **caractérisé en ce que** la dernière des chambres de traitement (K4) possède un dispositif de réflexion des UV (30 ; 32) sur sa face la plus éloignée de la source de rayonnement UV en direction de rayonnement.

16. Dispositif selon l'une ou plusieurs des revendications précédentes, **caractérisé en ce que** les éléments de tourbillonnement (28) du dispositif de prolongement du temps d'exposition sont réalisés transparents et perméables aux UV.

17. Dispositif selon l'une ou plusieurs des revendications précédentes, **caractérisé en ce que** la couche de séparation (T1 ; T2 ; T3 ; T4) transparente perméable aux UV présente des zones qui sont réalisées sous forme de lentille optique.

18. Dispositif selon l'une ou plusieurs des revendications précédentes, **caractérisé en ce que** des zones déformées en trois dimensions, d'une couche de séparation (T1 ; T2 ; T3 ; T4) transparente perméable aux UV, forment les éléments de tourbillonnement (28) transparents et perméables aux UV et réalisés sous forme de lentilles optiques ou de guides d'ondes optiques.

19. Dispositif selon l'une ou plusieurs des revendications précédentes, **caractérisé en ce qu'**un plan de sortie (ou plan d'entrée, respectivement) optique d'un élément de tourbillonnement (28) réalisé sous forme de lentille optique, d'une première couche de séparation (p.ex. T1), est agencé à une distance d'un plan d'entrée (ou plan de sortie, respectivement) optique d'un élément de tourbillonnement (28) réalisé sous forme de lentille optique, d'une deuxième couche de séparation (p.ex. T2) qui suit en direction de rayonnement (R), qui est inférieure ou égale à une distance qui est choisie parmi un groupe de distances comprenant : inférieure ou égale à 10 mm, inférieure ou égale à 9 mm, inférieure ou égale à 8 mm, inférieure ou égale à 7 mm, inférieure ou égale à 6 mm, inférieure ou égale à 5 mm, inférieure ou égale à 4 mm, inférieure ou égale à 3 mm, inférieure ou égale à 2 mm, inférieure ou égale à 1 mm, inférieure ou égale à 0,5 mm, inférieure ou égale à 0,25 mm.

20. Dispositif selon la revendication 19, **caractérisé en ce que** le plan d'entrée (ou plan de sortie, respectivement) optique d'un élément de tourbillonnement (28) réalisé sous forme de lentille optique, d'une couche de séparation (p.ex. T2) agencée de façon distale par rapport à la source de rayonnement UV en direction de rayonnement (R), est agencée à une distance du plan de sortie (ou plan d'entrée, respectivement) d'un élément de tourbillonnement (28) réalisé sous forme de lentille optique, d'une couche de séparation (p.ex. T1) agencée de façon proximale par rapport à la source de rayonnement UV en direction de rayonnement (R), qui est inférieure ou égale à une distance qui est choisie parmi un groupe de distances comprenant : inférieure ou égale à 10 mm, inférieure ou égale à 9 mm, inférieure ou égale à 8 mm, inférieure ou égale à 7 mm, inférieure ou égale à 6 mm, inférieure ou égale à 5 mm, inférieure ou égale à 4 mm, inférieure ou égale à 3 mm, inférieure ou égale à 2 mm, inférieure ou égale à 1 mm, inférieure ou égale à 0,5 mm, inférieure ou égale à 0,25 mm.

21. Dispositif selon l'une ou plusieurs des revendications précédentes, **caractérisé en ce que** celui-ci présente des couvercles supérieur (36) et inférieur (38) entre lesquels sont agencées la source de rayonnement UV (24) et les couches de séparation (T1 - T4) perméables aux UV, qui séparent les chambres de traitement (K1 - K4) les unes des autres, et **en ce qu'**au moins un des couvercles (36 ; 38) possède une admission (44) et/ou une évacuation (46) pour la substance à traiter, lesquelles communiquent avec au moins une première des chambres de traitement (K1 - K4).

22. Dispositif selon la revendication 21, **caractérisé en ce que** les couvercles (36, 38) présentent chacun un dispositif de centrage (40 ; 42 ; D1 - D3) pour les couches de séparation perméables aux UV qui sont agencées entre ceux-ci.

23. Dispositif selon la revendication 21 ou 22, **caractérisé en ce que** au moins un des couvercles (36, 38) présente au moins un canal de liaison (52) qui relie une chambre de traitement (K1 - K3) respective avec la chambre de traitement (K2 - K4) respectivement suivante.

24. Dispositif selon l'une des revendications 21 à 23, **caractérisé en ce qu'**au moins une partie (50, 52, D1 - D3) du dispositif de prolongement du temps d'exposition est agencée dans au moins un des couvercles (36, 38).

25. Dispositif selon l'une des revendications 21 à 24, **caractérisé en ce que** la partie du dispositif de prolongement du temps d'exposition, agencée dans au moins un des couvercles (36, 38), possède un dispositif de tourbillonnement (50, 52, D1 - D3) pour la substance à traiter.

26. Dispositif selon l'une des revendications 23 à 25, **caractérisé en ce que** le dispositif de tourbillonnement est intégré dans ledit au moins un canal de liaison (52).

27. Dispositif selon l'une ou plusieurs des revendications précédentes, **caractérisé en ce que** le dispositif de tourbillonnement présente un dispositif à buses (D1 - D3) avec au moins une buse (50).

28. Dispositif selon la revendication 27, **caractérisé en ce que** la buse (50) présente un orifice de sortie de buse débouchant dans une chambre de traitement (K1 - K4) qui, par rapport à la direction de rayonnement (R) de la source de rayonnement UV, définit une direction d'éjection de la substance à traiter, qui s'étend sous un angle oblique ou tangentiel et met en rotation la substance qui afflue dans la chambre de traitement (K 2 - K4) respective.

29. Dispositif selon la revendication 28, **caractérisé en ce que** les orifices de sortie de buse et les directions d'éjection des buses (50) de deux chambres de traitement (K1-K2 ; K2-K3 ; K3-K4) successives sont orientées dans des directions opposées les unes par rapport aux autres, de telle sorte que la direction de rotation de la substance s'inverse au passage d'une chambre de traitement à l'autre.

30. Dispositif selon la revendication 28 ou 29, **caractérisé en ce que** ladite au moins une buse (50) présente à son orifice de sortie de buse un point de discontinuité qui met la substance s'écoulant hors de la buse en tourbillons à haute fréquence.

31. Dispositif selon l'une ou plusieurs des revendications précédentes, **caractérisé en ce que** entre respectivement deux chambres de traitement (K1-K2 ; K2-K3 ; K3-K4) successives est prévue une multitude de buses (50 ; D1-D3) qui, par rapport à un axe longitudinal (L) des chambres de traitement (K1 - K4) ou à la source de rayonnement UV, sont agencées à la manière d'une couronne autour de l'axe longitudinal (L).

32. Dispositif selon l'une des revendications 23 à 31, **caractérisé en ce que** entre deux chambres de traitement (K1-K2 ; K2-K3 ; K3-K4) successives et reliées les unes autres par le canal de liaison (52) est agencé au moins un dispositif d'étranglement (50) pour la substance à traiter s'écoulant depuis une chambre de traitement vers la suivante, lequel (50) cause une montée de pression dans la substance et sollicite le dispositif de tourbillonnement (50 ; D1-D3) avec une pression prédéterminée de la substance à traiter.

33. Utilisation du dispositif selon l'une ou plusieurs des revendications 1 à 35 pour la combinaison de la stérilisation aux UV et de l'oxydation aux UV, en particulier pour le traitement des eaux.
